(19) Europäisches Patentamt
European Patent Office
Office européen des brevets

(11) **EP 3 597 808 B1**

(12) **EUROPEAN PATENT SPECIFICATION**

(45) Date of publication and mention
of the grant of the patent:
**08.09.2021 Bulletin 2021/36**

(51) Int Cl.:
***A61F 13/511*** *(2006.01)*   ***B32B 1/00*** *(2006.01)*

(21) Application number: **17901274.5**

(86) International application number:
**PCT/JP2017/010464**

(22) Date of filing: **15.03.2017**

(87) International publication number:
**WO 2018/167881 (20.09.2018 Gazette 2018/38)**

(54) **LAMINATED NONWOVEN FABRIC, METHOD FOR MANUFACTURING SAME, ABSORPTIVE ARTICLE, AND METHOD FOR ABSORBING SWEAT**

LAMINIERTER VLIESSTOFF, VERFAHREN ZUR HERSTELLUNG DAVON, SAUGFÄHIGER ARTIKEL UND VERFAHREN ZUM ABSORBIEREN VON SCHWEISS

TISSU NON TISSÉ STRATIFIÉ, SON PROCÉDÉ DE FABRICATION, ARTICLE ABSORBANT ET PROCÉDÉ POUR ABSORBER LA TRANSPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.01.2020 Bulletin 2020/04**

(73) Proprietor: **Kao Corporation
Chuo-ku
Tokyo 103-8210 (JP)**

(72) Inventors:
• **MINATOZAKI, Masayuki
Tochigi 321-3497 (JP)**
• **KOBAYASHI, Hideyuki
Tochigi 321-3497 (JP)**
• **FUKUDA, Yuko
Tochigi 321-3497 (JP)**

(74) Representative: **Vossius & Partner
Patentanwälte Rechtsanwälte mbB
Siebertstrasse 3
81675 München (DE)**

(56) References cited:
**WO-A1-2006/011564   JP-A- 2002 065 738
JP-A- 2007 130 293   JP-A- 2007 181 662
JP-A- 2010 063 934   JP-A- 2011 144 480**

**Description**

Technical Field

The present invention relates to a laminated nonwoven fabric having a laminated structure in which a plurality of fibre layers are stacked, and also relates to an absorbent article and a sweat absorbing sheet using the laminated nonwoven fabric.

Background Art

Typical absorbent articles, such as nappies and sanitary towels, employ nonwoven fabrics having a laminated structure of two or more layers, nonwoven fabrics whose surfaces have concavo-convex shapes, and so forth, as constituents. JP 2016-112167 A, for example, describes that a sweat absorbing sheet capable of absorbing sweat of a user is disposed on a portion of a nappy that contacts the skin of a user, and also describes that a sweat absorbing sheet having a structure in which an elastic member is disposed between two nonwoven fabric sheets is used as the sweat absorbing sheet. One of the two nonwoven fabric sheets constituting the sweat absorbing sheet of JP 2016-112167 A located closer to the skin of the user has a lower hydrophilic degree than the other nonwoven fabric sheet located relatively away from the skin of the user. The sweat absorbing sheet of JP 2016-112167 A is obtained by supplying a molten elastomer resin to space between two nonwoven fabric sheets in a strand-like shape or a sheet-like shape and then uniting the elastomer resin and the nonwoven fabric sheets. This sweat absorbing sheet does not have depressions where layers constituting the sweat absorbing sheet are joined together, that is, depressions formed by a process of heating an object while compressing the object in the thickness direction (heat embossing).

JP 2006-51649 A describes, as a laminated nonwoven fabric suitable for a constituent of an absorbent article, a unidirectionally water-permeable nonwoven fabric sheet having a liquid permeability in one surface direction and having no liquid permeability in the opposite direction, and also describes, as an embodiment of the unidirectionally water-permeable nonwoven fabric sheet, a nonwoven fabric in which at least one layer is hydrophilised and the other layers are not hydrophilised. JP 2006-51649 A also describes, as a method for manufacturing a laminated nonwoven fabric, a method of performing a fusion bonding process by heat embossing rollers on stacked nonwoven fabric sheets, and also describes, as another manufacturing method, a method of depositing long fibres having a given fineness directly on a spunbond nonwoven fabric and then performing either an entangling process with a means such as needle punching, water jetting, ultrasonic sealing, or a fusion bonding process with heat embossing rollers.

JP 2002-233720 A describes, as a functional filter for use in extracting coffee and tea, a laminated nonwoven fabric in which an inner layer of a hydrophobic microfibre nonwoven fabric having an average fibre diameter of 0.1 through 6 $\mu$m, an outer layer of a synthetic fibre nonwoven fabric to which a hydrophilic agent is attached, and an intermediate layer interposed between the inner layer and the outer layer and formed of a synthetic fibre nonwoven fabric having an average fibre diameter of 10 through 100 $\mu$m, and are partially joined to one another by an adhesive or heat embossing.

EP 1 184 020 A2 discloses absorbent article including: a liquid permeable surface member; a backing sheet; and an absorbent layer interposed between the surface member and the backing sheet. The surface member includes an upper layer located at a liquid-receiving side surface and a lower layer located adjacent to the absorbent layer. The upper layer is formed of first continuous filaments. The lower layer is formed of second continuous filaments. The first and second continuous filaments individually extend over the entire length of the surface member. Hydrophilicity of the lower layer is higher than that of the upper layer.

Summary of Invention

The present invention provides a laminated nonwoven fabric having a laminated structure of fibre layers including long fibres as defined in claim 1.

The present invention also provides a method for manufacturing a laminated nonwoven fabric having a laminated structure of fibre layers including long fibres in which the layers constituting the laminated structure are joined to one another in an interlayer fused portion, as defined in independent claim 8

The present invention also provides an absorbent article including the laminated nonwoven fabric according to the present invention described above. In the absorbent article, the laminated nonwoven fabric is disposed with the second surface facing skin of a user.

The present invention also provides a sweat absorbing sheet capable of absorbing sweat including the laminated nonwoven fabric according to the present invention described above.

Brief Description of Drawings

[Fig. 1] Fig. 1 is a cross-sectional view schematically illustrating a cross section of an embodiment of a laminated nonwoven fabric according to the present invention, taken along a thickness direction.

[Fig. 2] Fig. 2 is a view schematically illustrating a liquid absorbing mechanism in the laminated nonwoven fabric illustrated in Fig. 1.

[Fig. 3] Figs. 3(a) through 3(h) are views each schematically illustrating a pattern of an interlayer fused portion according to the present invention.

[Fig. 4] Fig. 4 is a schematic drawing of an embodiment of a method for manufacturing a laminated nonwoven fabric according to the present invention.

[Fig. 5] Fig. 5 is a schematic drawing of another embodiment of a method for manufacturing a laminated nonwoven fabric according to the present invention.

[Fig. 6] Fig. 6 is a schematic perspective view of a pull-on nappy as an embodiment of an absorbent article according to the present invention.

[Fig. 7] Fig. 7 is a developed plan view schematically illustrating a skin-facing surface side (inner surface side) of the nappy illustrated in Fig. 6 in an unfolded and stretched state.

[Fig. 8] Fig. 8 is a vertical cross-sectional view schematically illustrating a cross section taken along line I-I in Fig. 7.

Description of Embodiments

In an absorbent article, a nonwoven fabric used as a constituent (e.g., a topsheet) disposed relatively close to the skin of a user is required to absorb body fluid quickly such as sweat and urine, to diffuse the absorbed body fluid efficiently in a surface direction orthogonal to the thickness direction of the nonwoven fabric and to be dried quickly. As such a quick-dried nonwoven fabric, a laminated nonwoven fabric having different affinities for water between the front and back surfaces has been known, as described in JP 2016-112167 A. Here, a possible method for making the hydrophilic degree vary in the thickness direction of the nonwoven fabric includes a method of making the amount of use of a hydrophilising agent nonuniform in the thickness direction, but this method alone has a room for improvement in order to sufficiently satisfy the requirement.

A possible method for manufacturing a laminated nonwoven fabric in which the hydrophilic degree is different between the front and the back also includes a method of conveying a base nonwoven fabric and spinning another fibres having a hydrophilic degree different from that of constituent fibres of the base nonwoven fabric to deposit these fibres on the nonwoven fabric being conveyed in a web form, and then performing heat embossing on the deposits of the fibres to unite the deposits. This method, however, needs a mechanism for additionally producing a base nonwoven fabric and conveying the fabric with stability, and thus, is disadvantageous in terms of manufacturing costs, and conveyance stability of the base nonwoven fabric, for example.

Thus, the present invention relates to providing a laminated nonwoven fabric having high absorption performance for body fluids such as sweat and urine, a manufacturing method for the laminated nonwoven fabric, an absorbent article, and a sweat absorbing sheet.

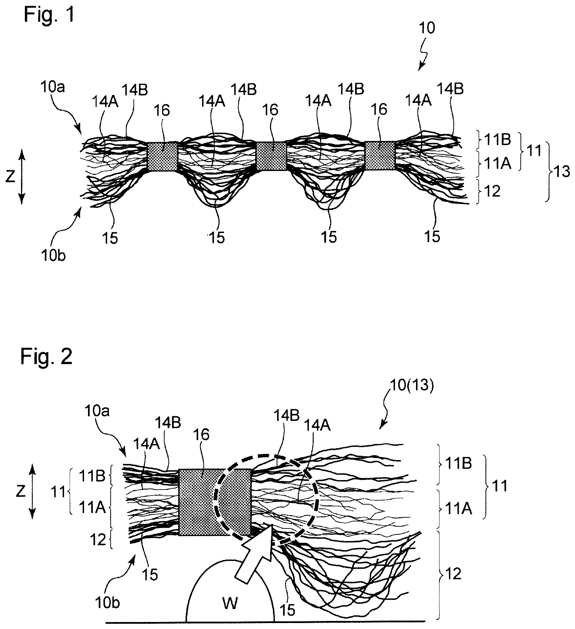

The present invention will now be described with reference to the drawings based on preferred embodiments thereof. Fig. 1 schematically illustrates a cross section of a laminated nonwoven fabric 10 as one embodiment of a laminated nonwoven fabric according to the present invention, taken along a thickness direction Z thereof. The laminated nonwoven fabric 10 has a laminated structure 13 of fibre layers including long fibres (thermoplastic fibres). The fibre layers are typically layers constituting a nonwoven fabric, that is, a single-layer nonwoven fabric (e.g., spunbond nonwoven fabric) or a laminated nonwoven fabric (e.g., SMS nonwoven fabric). The laminated structure 13 includes a first surface 10a that is a surface (outer surface) of the laminated nonwoven fabric 10 and a second surface 10b that is the other surface (outer surface) of the laminated nonwoven fabric 10 and located opposite to the first surface 10a.

A main feature of the laminated nonwoven fabric 10 is that the laminated structure 13 has a gradient of a hydrophilic degree (hydrophilic degree gradient) in the thickness direction Z. That is, in the laminated structure 13, the second surface 10b is constituted by a hydrophobic layer 12 including hydrophobic fibres 15, a hydrophilic layer 11 including hydrophilic fibres 14A and 14B is disposed at the first surface 10a side of the hydrophobic layer 12, and the hydrophilic layer 11 includes a first hydrophilic layer 11A and a second hydrophilic layer 11B disposed in this order from the hydrophobic layer 12. This configuration provides the laminated structure 13 with a hydrophilic degree gradient in which "hydrophilic degree is relatively low at the second surface 10b and increases from the second surface 10b inward in the thickness direction Z."

In the laminated nonwoven fabric 10 illustrated in Fig. 1, the laminated structure 13 has a triple-layer structure including the hydrophilic layer 11, having a double-layer structure of the first hydrophilic layer 11A and the second hydrophilic layer 11B, and the hydrophobic layer 12. The first surface 10a is formed of the second hydrophilic layer 11B and is hydrophilic. The second surface 10b is formed of the hydrophobic layer 12 and is hydrophobic. Here, the expression "the number of layers of the laminated structure 13 is three" refers to the total number of layers of three layers having different shapes or functions (i.e., the first hydrophilic layer 11A, the second hydrophilic layer 11B, and the hydrophobic layer 12), and does not strictly correspond to the number of layers of fibre layers (nonwoven fabric). That is, each of the layers 11A, 11B, and 12 has not only a single-layer structure but can also have a multi-layer structure of two or more layers. If each of the hydrophilic layers 11A and 11B is a fibre layer having a single-layer structure, and the hydrophobic layer 12 is a fibre layer having a triple-layer structure, although the laminated structure 13 has a triple-layer structure including the layers 11A, 11B, and 12, the actual number of fibre layers is five in total, specifically, one layer in the first hydrophilic layer 11A, one layer in the second hydrophilic layer 11B, and three layers in the hydrophobic layer 12. That is, each of the hydrophilic layers 11A and 11B and the hydrophobic layer 12 can be of two or more layers.

The first hydrophilic layer 11A is constituted mainly by the hydrophilic fibres 14A, and thus, is a hydrophilic layer. The second hydrophilic layer 11B is constituted mainly by the hydrophilic fibres 14B, and thus, is a hydrophilic layer. The hydrophobic layer 12 is constituted mainly by the hydrophobic fibres 15, and thus, is a hydrophobic layer. The first hydrophilic layer 11A contains at least 50 mass % of the hydrophilic fibres 14A with respect to the total mass of the first hydrophilic layer 11A, and the content of the hydrophilic fibres 14A may be 100 mass %. The second hydrophilic layer 11B contains at least 50 mass % of the hydrophilic fibres 14B with respect to the total mass of the second hydrophilic layer 11B, and the content of the hydrophilic fibres 14B may be 100 mass %. The hydrophobic layer 12 contains at least 70 mass % of the hydrophobic fibres 15 with respect to the total mass of the hydrophobic layer 12, and the content of the hydrophobic fibres 15 may be 100 mass %.

In the present invention, a hydrophilic degree of fibres is determined based on a contact angle with water measured by a method described later. If the contact angle is less than 90 degrees, the fibres are hydrophilic, and if the contact angle is 90 degrees or more, the fibres are hydrophobic. As the contact angle with water measured by the method below decreases, the hydrophilic degree increases (the hydrophobic degree decreases), and as the contact angle increases, the hydrophilic degree decreases (the hydrophobic degree increases). In the laminated nonwoven fabric 10, the contact angle measured by the method described below is less than 90 degrees in each of the hydrophilic fibres 14A and 14B constituting the hydrophilic layers 11A and 11B of the laminated structure 13, and the contact angle measured by the method described below in the hydrophobic fibres 15 constituting the hydrophobic layer 12 is 90 degrees or more.

<Method for measuring contact angle>

Fibres are taken from a measurement target (laminated nonwoven fabric), and the contact angle of water with the fibres is measured. In taking fibres, scissors and tweezers are used. The fibres are taken from portions of a laminated nonwoven fabric as the measurement target, specifically, from the outermost surface (external surface) of each of the first surface and the second surface and a region of the laminated nonwoven fabric sandwiched between the first surface and the second surface. As a measurement device, an automatic contact angle meter MCA-J manufactured by Kyowa Interface Science Co., Ltd. is used. Measurement of a contact angle uses deionised water. The amount of liquid discharged from an ink jet water drop discharge part (pulse injector CTC-25 manufactured by Cluster Technology Co., Ltd. and having a discharge port diameter of 25 $\mu$m) is set at 15 picolitres, and water drops are dropped directly onto fibres. The state of this drop is recorded by a high-speed recording device connected to a camera oriented horizontally. The recording device is preferably a personal computer incorporating a high-speed capture device, from the viewpoint of a subsequent image analysis. In this measurement, an image is recorded at every 17 msec. In recorded images, a first image in which water drops fall on fibres is subjected to an image analysis with a furnished software FAMAS (version of the software: 2.6.2, analysis technique: drop method, analysis method: $\theta$/2 method, image processing algorithm: nonreflection, image processing image mode: frame, threshold level: 200, and no curvature correction), and an angle formed by a surface of water drops contacting the air and fibres is calculated and is used as a contact angle. Fibres taken from a measurement object are cut into a fibre length of 1 mm, and the resulting fibres are placed on a sample base of the contact angle meter and are held horizontally. Contact angles are measured at two different points for one fibre. Contact angles of N=5 fibres are measured to the first decimal place, and an average of values measured at ten points in total (rounded to the first decimal place) is defined as a contact angle of these fibres with water. Measurement environments are a room temperature of 22±2°C, and a humidity of 65±2%RH As the contact angle decreases, the hydrophilic degree increases.

In a case where a measurement sample (e.g., fibres) is included in a constituent of an absorbent article (e.g., a topsheet or a sweat absorbing sheet), as a method for extracting a measurement sample, if the constituent including the measurement sample is fixed to another constituent by an adhesive or by fusing, for example, this fixture needs to be cancelled and a method for taking the constituent including the measurement sample from the absorbent article needs to be employed. However, if the constituent including the measurement sample is not fixed to another constituent, a method of extracting the measurement sample directly from the absorbent article can be employed. As a method for cancelling fixing of the constituent, it is preferable to employ a method in which an adhesive or the like used for joining the constituent of the measurement target to another constituent in the absorbent article is weakened with a cooling means such as a cold spray and then the constituent of the measurement target is carefully peeled off to be extracted. This extraction method is applied to a measurement of a measurement target according to the present invention, such as measurements of an inter-fibre distance and a fineness described later. From the viewpoint of minimising the influence on a hydrophilising agent applied to a constituent, it is preferable not to employ a method that might cause deterioration or a loss of an oil agent, such as application of a solvent or blowing of hot air with a dryer, as a method for removing a fixing portion.

The hydrophobic fibres 15 may be hydrophobic thermoplastic fibres (fusion-bonding fibres). Examples of a material for the hydrophobic fibres 15 as hydrophobic thermoplastic fibres include: polyolefins such as polyethylene and polypropylene; polyesters such as polyethylene terephthalate; polyamides such as nylon 6 and nylon 66; polyacrylic acid, poly(alkyl methacrylate), polyvinyl chloride, and polyvinylidene chloride. Each of these materials may be used alone or two or more of the materials may be used in combination.

On the other hand, the hydrophilic fibres 14A and 14B may be hydrophilic thermoplastic fibres (fusion-bonding fibres). Specifically, the hydrophilic fibres 14A and 14B may be inherently hydrophilic thermoplastic fibres such as polyacrylonitrile fibres, or may be hydrophobic thermoplastic fibres usable as the hydrophobic fibres 15 and subjected to a hydrophilic treatment. Each of these materials may be used alone or two or more of the materials may be used in combination. Examples of the latter "thermoplastic fibre subjected to a hydrophilic treatment" include thermoplastic fibres in which a hydrophilising agent is kneaded, thermoplastic fibres having surfaces to which a hydrophilising agent is attached, and thermoplastic fibres subjected to a plasma treatment. The hydrophilising agent may be any typical hydrophilising agent used for sanitary applications.

The hydrophilic fibres 14A that are main constituent fibres of the first hydrophilic layer 11A and the hydrophilic fibres 14B that are main constituent fibres of the second hydrophilic layer 11B may have the same properties such as a fibre diameter and a hydrophilic degree or may have different properties. As will be described later, a difference in hydrophilic degree between the first hydrophilic layer 11A and the second hydrophilic layer 11B can enhance properties of the laminated nonwoven fabric 10, such as liquid absorbency. Thus, the hydrophilic fibres 14A and the hydrophilic fibres 14B are typically different from each other.

Each of the hydrophilic fibres 14A and 14B and the hydrophobic fibres 15 may be single fibres constituted by one type of a synthetic resin (thermoplastic resin) or blended polymers as a mixture of two or more types of synthetic resins, or may be bicomponent fibres. The bicomponent fibres here refer to fibres that are synthetic fibres (thermoplastic fibres) obtained by combining two or more types of synthetic resins (thermoplastic resins) having different components with a spinneret, and spinning the fibres at the same time and that have a structure in which a plurality of components are continuous in the length direction of the fibres, and are bonded to one another in single fibres. The form of the bicomponent fibres can be a sheath-core type or a side-by-side type, and is not limited to a specific form.

Each of the hydrophilic layer 11 (the first hydrophilic layer 11A and the second hydrophilic layer 11B) and the hydrophobic layer 12 constituting the laminated structure 13 is a fibre layer mainly constituted by long fibres, and is a nonwoven fabric (long fibre nonwoven fabric) in the laminated nonwoven fabric 10. The term "mainly constituted by" means that the proportion of long fibres in the total mass of the nonwoven fabric is 70 mass % or more, and the proportion is typically 100 mass %.

In the present invention, "long fibres" refer to fibres having a fibre length of 30 mm or more. In particular, so-called continuous long fibres having a fibre length of 150 mm or more are preferable because a long fibre nonwoven fabric having a high breaking strength can be obtained. The upper limit of the fibre length in the "long fibres" is not specifically limited. The "long fibre nonwoven fabric" typically refers to a nonwoven fabric including a fibre aggregate in which long fibres are intermittently fixed with fusion-bonded portions. In general, a long fibre nonwoven fabric has a strength higher than a nonwoven fabric mainly constituted by short fibres (short fibre nonwoven fabric), such as an air-through nonwoven fabric. Examples of such a long fibre nonwoven fabric include a single-layer nonwoven fabric such as a spunbond nonwoven fabric and a meltblown nonwoven fabric, a laminated nonwoven fabric in which spunbond layers mainly constituted by long fibres or meltblown layers, for example, are stacked, and a heat roll nonwoven fabric formed by a card method. Examples of the laminated nonwoven fabric include a spunbond-spunbond laminated nonwoven fabric (SS nonwoven fabric), a spunbond-spunbond-spunbond laminated nonwoven fabric (SSS nonwoven fabric), a spunbond-meltblown-spunbond laminated nonwoven fabric (SMS nonwoven fabric), and a spunbond-meltblown-meltblown-spunbond nonwoven fabric (SMMS nonwoven fabric).

As another main feature of the laminated nonwoven fabric 10 is that the laminated nonwoven fabric 10 includes a "high-density fused portion" in which constituent fibres are arranged at a density higher than those in peripheral portions and are fused to one another. The term "at a density higher than those in peripheral portions" can also be expressed as "with a thickness smaller than those in peripheral portions." That is, the laminated structure 13 includes interlayer fused portions 16 in which layers constituting the laminated structure 13 are arranged at a density higher than those in peripheral portions and fused to one another. In the interlayer fused portions 16, constituent fibres of the layers constituting the laminated structure 13 (i.e., the first hydrophilic layer 11A, the second hydrophilic layer 11B, and the hydrophobic layer 12 in the illustrated example) are fusion-bonded to one another and joined together by fusing. In the laminated structure 13 having such a structure, the interlayer fused portions 16 are formed in the same pattern in both of the first surface 10a and the second surface 10b.

The interlayer fused portions 16 are densified portions in which constituent fibres in these portions are densified in the thickness direction, and this densification is typically performed by embossing with a melting promotion means for promoting melting of thermoplastic fibres as constituent fibres, such as heat or ultrasonic waves, specifically performed by, for example, heat sealing or ultrasonic sealing. When focused on such a manufacturing method, the interlayer fused portions 16 can also be referred to as embossed portions or compressed portions, for example.

In the laminated nonwoven fabric 10, the laminated structure 13 does not include a high-density fused portion, that is, a portion in which constituent fibres are arranged at a density higher than those in peripheral portions and fused to one another, except for the interlayer fused portions 16.

In addition, in the laminated nonwoven fabric 10, layers constituting the laminated structure 13, more specifically, the hydrophobic layer 12, the first hydrophilic layer 11A, and the second hydrophilic layer 11B, are joined together only by heat fusion bonding of constituent fibres.

As described above, the laminated nonwoven fabric 10 has features in which: (1) the laminated structure 13 has a hydrophilic degree gradient in the thickness direction Z; and (2) layers constituting the laminated structure 13 (i.e., the hydrophilic layer 11 and the hydrophobic layer 12) are joined together in the interlayer fused portions 16. These features can enhance performance of absorbing body fluid such as sweat and urine.

That is, in the laminated nonwoven fabric 10, the second surface 10b, which is constituted by the hydrophobic layer 12 including the hydrophobic fibres 15, basically has difficulty in absorbing body fluid such as sweat and urine (aqueous liquid). In the interlayer fused portions 16 of the second surface 10b and their peripheral portions, as illustrated in Fig. 2, not only the hydrophobic fibres 15 but also the hydrophilic fibres 14A of the first hydrophilic layer 11A that is a layer adjacent to the hydrophobic layer 12 and has a higher hydrophilic degree are relatively closely arranged. Thus, the proportion of the hydrophilic fibres 14A is higher than those in other portions of the second surface 10b, and accordingly, body fluid W can be attached to the interlayer fused portions 16 and their peripheral portions by priority in the second surface 10b. In addition, as described above, the laminated structure 13 has a hydrophilic degree gradient in the thickness direction Z in which "hydrophilic degree is relatively low at the second surface 10b and increases from the second surface 10b inward in the thickness direction Z." Thus, the laminated nonwoven fabric 10 has a high liquid absorbency from the second surface 10b inward in the thickness direction Z, and has a strong capillary force. Accordingly, body fluid W attached to the interlayer fused portions 16 and their peripheral portions in the second surface 10b is quickly drawn mainly from peripheral portions of the interlayer fused portions 16 and their vicinities into the laminated nonwoven fabric 10, and while being diffused in the surface direction of the laminated nonwoven fabric 10 (direction orthogonal to the thickness direction Z), moves inward in the thickness direction Z, and is absorbed and held in the hydrophilic layer 11 (the first hydrophilic layer 11A and the second hydrophilic layer 11B). In the second surface 10b, portions into which liquid is drawn are mainly "peripheral portions of the interlayer fused portions 16 and their vicinities." In general, even if the interlayer fused portions 16 are changed into a film form because of a loss of a fibre form of constituent fibres in centre portions thereof, portions of the interlayer fused portions 16 except the centre portions, that is, peripheral portions of the interlayer fused portions 16 and their vicinities (i.e., portions around the interlayer fused portions 16), are not changed into a film form and the fibre form is maintained. Thus, the peripheral portions of the interlayer fused portions 16 and their vicinities serve as liquid drawing portions in the second surface 10b.

From the viewpoint of further enhancement of liquid absorbency by the hydrophilic degree gradient, especially wide diffusion of liquid taken from the hydrophobic layer 12 (second surface 10b) and transferred to the first hydrophilic layer 11A in a surface direction of the laminated nonwoven fabric 10, the second hydrophilic layer 11B preferably has a hydrophilic degree of constituent fibres higher than that of the first hydrophilic layer 11A, that is, preferably has a contact angle measured by the method described above smaller than that of the first hydrophilic layer 11A. The hydrophilic degree of constituent fibres can be adjusted by appropriately adjusting the degree of a hydrophilic treatment of long fibres (thermoplastic fibres) that are main constituent fibres of the laminated nonwoven fabric 10, for example, the type and/or the content of a hydrophilising agent, for example.

Based on the premise that the contact angle of constituent fibres (hydrophobic fibres 15) of the hydrophobic layer 12 is larger than that of the hydrophilic layer 11 (the first hydrophilic layer 11A and the second hydrophilic layer 11B), in the case of using the laminated nonwoven fabric 10 as an absorbent article or a sweat absorbing sheet, from the viewpoint of reducing the amount of liquid attached to the skin, the contact angle of constituent fibres (hydrophobic fibres 15) of the hydrophobic layer 12 is preferably 90 degrees or more and more preferably 95 degrees or more, and preferably 150 degrees or less and more preferably 145 degrees or less.

Based on the premise that the contact angle of constituent fibres (hydrophilic fibres 14A) of the first hydrophilic layer 11A is smaller than that of the hydrophobic layer 12 and larger than that of the second hydrophilic layer 11B, in the case of using the laminated nonwoven fabric 10 as an absorbent article or a sweat absorbing sheet, from the viewpoint of enhancing a force of drawing liquid attached to the skin into the first hydrophilic layer 11A through the hydrophobic layer 12, the contact angle of constituent fibres (hydrophilic fibres 14A) of the first hydrophilic layer 11A is preferably 60 degrees or more and more preferably 65 degrees or more, and preferably 88 degrees or less and more preferably 85 degrees or less.

Based on the premise that the contact angle of constituent fibres (hydrophilic fibres 14B) of the second hydrophilic layer 11B is smaller than those of constituent fibres (the hydrophobic fibres 15 and the hydrophilic fibres 14A) of the hydrophobic layer 12 and the second hydrophilic layer 11B, in the case of using the laminated nonwoven fabric 10 as an absorbent article or a sweat absorbing sheet, from the viewpoint of enhancing a force of drawing liquid that has drawn into the first hydrophilic layer 11A toward the first surface 10a and the viewpoint of widely diffusing liquid in the surface direction of the laminated nonwoven fabric 10 for an increase in exhalation property, the contact angle of constituent fibres (hydrophilic fibres 14B) of the second hydrophilic layer 11B is preferably 15 degrees or more and more preferably 20 degrees or more, and preferably 80 degrees or less and more preferably 75 degrees or less.

In the case of using the laminated nonwoven fabric 10 as an absorbent article or a sweat absorbing sheet, from the viewpoint of obtaining both permeability of liquid in the thickness direction and resistance to liquid back, a difference $(V1-V2)$ between the contact angle $(V1)$ of constituent fibres (hydrophobic fibres 15) of the hydrophobic layer 12 and the contact angle $(V2)$ of constituent fibres (hydrophilic fibres 14B) of the first hydrophilic layer 11A is preferably 3 degrees or more and more preferably 5 degrees or more, and preferably 30 degrees or less and more preferably 25 degrees or less, based on the premise that $V1 > V2$. By adjusting the difference to the lower limit or more, liquid is drawn from the hydrophobic layer 12 to the first hydrophilic layer 11A so that attachment of liquid to the skin can be suitably reduced. The difference $V1 - V2$ is preferably as large as possible, basically, but from the viewpoints of appropriately reducing liquid retainability in the first hydrophilic layer 11A and obtaining suitable permeability to the second hydrophilic layer 11B, the contact angle is preferably set to the upper limit described above or less.

In the case of using the laminated nonwoven fabric 10 as an absorbent article or a sweat absorbing sheet, from the viewpoints of obtaining all of liquid permeability in the thickness direction, a liquid diffusion property in the surface direction, and resistance to liquid back, a difference $(V2 - V3)$ between the contact angle $(V2)$ of constituent fibres (hydrophilic fibres 14B) of the first hydrophilic layer 11A and the contact angle $(V3)$ of constituent fibres (hydrophilic fibres 14B) of the second hydrophilic layer 11B is preferably 3 degrees or more and more preferably 5 degrees or more, and preferably 30 degrees or less and more preferably 25 degrees or less, based on the premise that $V2 > V3$. By adjusting the difference to the lower limit or more, liquid is drawn from the first hydrophilic layer 11A into the second hydrophilic layer 11B so that a high diffusion property of liquid in the surface direction of the laminated nonwoven fabric 10 and a high exhalation property can be obtained. The difference $V2 - V3$ is preferably as large as possible, basically, and from the viewpoints that liquid retainability in the second hydrophilic layer 11B appropriately reduces an exhalation property and suitably suppresses liquid back to the first surface 10a to enhance vapourisability, the difference is preferably set to the upper limit described above or less.

As described above, a difference is preferably present between the contact angles of constituent fibres in two of the layers 11A, 11B, and 12 constituting the laminated structure 13 of the present invention, that is, affinities for water of two types of constituent fibres are preferably different, more specifically, the contact angles measured by the method described above are preferably different from each other by three degrees or more.

In addition to the two features described above, the laminated nonwoven fabric 10 has a feature (3) in which an inter-fibre distance of constituent fibres of the first hydrophilic layer 11A is shorter than that of the hydrophobic layer 12. The laminated nonwoven fabric 10 having all the features (1) through (3) shows excellent absorption performance of body fluid such as sweat and urine.

A relationship between the feature (3) and advantages thereof is as follows:

That is, liquid absorbency of the laminated nonwoven fabric 10 is affected not only by the hydrophilic degree gradient in the thickness direction described above but also by a density gradient of fibres in the thickness direction more or less. As illustrated in Fig. 2, in a case where the hydrophobic layer 12 (second surface 10b) is a layer that first contacts liquid to be absorbed (layer that draws liquid therein), to increase liquid absorbency of the laminated nonwoven fabric 10, the density preferably increases from the hydrophobic layer 12 (second surface 10b) inward in the thickness direction Z of the laminated structure 13. That is, the hydrophobic layer 12 preferably has a relatively small density (long inter-fibre distance), and its adjacent layer (the first hydrophilic layer 11A in the illustrated example) preferably has a relatively large density (short inter-fibre distance). With this configuration, a capillary force of the laminated nonwoven fabric 10 increases so that liquid is quickly drawn to the inside from the interlayer fused portions 16 in the second surface 10b and their peripheral portions, and the drawn liquid can be absorbed and retained in the hydrophilic layer 11 (the first hydrophilic layer 11A and the second hydrophilic layer 11B) with further stability.

As described above, to enhance liquid absorbency by providing the laminated nonwoven fabric 10 with a density gradient in which the density increases from the hydrophobic layer 12 (second surface 10b) inward in the thickness direction Z of the laminated structure 13, the fibre diameter of constituent fibres of the hydrophobic layer 12 is relatively large so that the inter-fibre distance of constituent fibres of the hydrophobic layer 12 is relatively long, and the fibre diameter of constituent fibres of the first hydrophilic layer 11A adjacent to the hydrophobic layer 12 is relatively small so that the inter-fibre distance of constituent fibres of the first hydrophilic layer 11A is relatively short. The fibre diameter and the inter-fibre distance are measured by the following methods, respectively.

<Method for measuring fibre diameter>

A measurement target (fibre layer, laminated nonwoven fabric) is cut with a razor (e.g., single-edged razor manufactured by FEATHER SAFETY RAZOR Co., Ltd.), thereby obtaining a sample piece having a rectangular shape in plan view (8 mm × 4 mm). The measurement target is carefully cut not to damage the structure of a cutting plane of the sample piece formed by the cutting, with, for example, a pressure in cutting. Examples of preferred methods for cutting the measurement target include a method in which the measurement target is placed in liquid nitrogen to be sufficiently frozen before cutting of the measurement target and then the measurement target is cut. Using a double-sided paper tape (NICE TACK NW-15 manufactured by Nichiban Co., Ltd.), a sample piece is bonded to a sample table. Next, the sample piece is coated with platinum. The coating employs an ion spattering device E-1030 (product name) manufactured by Hitachi Naka Seiki Kabushiki Kaisha, and the spattering time is 30 seconds. A cross section of the sample piece is observed with an S-4000 field-emission scanning electron microscope manufactured by Hitachi, Ltd. with a 1000-fold magnification. For example, in a case where the measurement target is a laminated nonwoven fabric having a laminated structure similar to that of the laminated structure 13, from an electron microscopy image, boundaries between layers of the laminated nonwoven fabric is determined based on a difference in fibre diameter and/or density, lateral lengths of fibres relative to the longitudinal direction of fibres are measured for 10 fibres out of each of fibres (hydrophobic fibres 15) facing one surface (second surface 10b), fibres (hydrophilic fibres 14A) having a fibre diameter smaller than that of the fibres facing the surface (second surface 10b), and fibres (hydrophilic fibres 14B) disposed at the other surface (first surface 10a), and the average of these lengths is used as a fibre diameter.

<Method for measuring inter-fibre distance>

An inter-fibre distance of a fibre aggregate such as a nonwoven fabric or paper is obtained by Equation (1) below based on the assumption by Wrotnowski. Equation (1) is typically used for obtaining an inter-fibre distance of a fibre aggregate. On the assumption by Wrotnowski, fibres are cylindrical, and are regularly arranged without an intersection.

In a case where a sheet of a measurement target (the first hydrophilic layer 11A, the second hydrophilic layer 11B, and the hydrophobic layer 12) has a single-layer structure, an inter-fibre distance of the sheet having the single-layer structure can be obtained by Equation (1).

In a case where a sheet of the measurement target (the first hydrophilic layer 11A, the second hydrophilic layer 11B, and the hydrophobic layer 12) has a multi-layer structure, as in the case of an SMS nonwoven fabric, an inter-fibre distance of the sheet having the multi-layer structure can be obtained in the following procedure.

First, an inter-fibre distance of each fibre layers constituting a multi-layer structure is calculated from Equation (1) below. In this calculation, as a thickness t, a basis weight W, a fibre resin density p, and a fibre diameter D used in Equation (1), those used for layers of the measurement target are used. Each of the thickness t, the basis weight W, and the fibre diameter D is an average of values measured at a plurality of measurement points.

The thickness t (mm) is measured in the following manner. First, a sheet as a measurement target is cut into 50 mm in longitudinal direction ×50 mm in width direction, thereby producing a cut piece of the sheet. In a case where a cut piece of this size cannot be produced as a sheet of a measurement target, for example, a case where a sheet is taken from a small absorbent article, a cut piece that is as large as possible is produced. Subsequently, the cut piece is placed on a flat plate, a flat glass plate is then placed on the flat plate, and a weight is uniformly placed on the glass plate such that a load including the glass plate is 49 Pa, and a thickness of the cut piece is measured. As measurement environments, the temperature is $20 \pm 2°C$, the relative humidity is $65 \pm 5\%$, and the measurement equipment is a microscope (manufactured by KEYENCE CORPORATION, VHX-1000). To measure the thickness of the cut piece, first, an enlarged photograph of a cutting plane of the cut piece is obtained. The enlarged photograph also includes an object having known dimensions. Next, a scale is aligned with the enlarged photograph of the cutting plane of the cut piece, and a thickness of the cutting piece, that is, a thickness of a measurement target sheet, is measured. The foregoing process is repeated three times, and the average of the three processes is used as a thickness t of the measurement target sheet. In a case where the measurement target sheet is a laminated product, boundaries between layers are determined based on a difference in fibre diameter and/or a density, and a thickness is calculated.

The basis weight W $(g/m^2)$ is obtained by cutting the measurement target sheet into a predetermined size (e.g.,

12 cm × 6 cm), and after measurement of a mass, dividing the measured mass by an area obtained from the predetermined size ("basis weight W (g/m$^2$)= mass ÷ area obtained by predetermined size"). The measurement is performed four times, and the average is used as the basis weight.

The fibre resin density ρ (g/cm$^3$) is measured based on a measuring method of a density gradient tube method described in a JIS L1015 chemical fibre staple test method using a density gradient tube (URL http://kikakurui.com/l/L1015-2010-01.html, document: JIS Handbook Fibre - 2000, Japanese Standards Association, pp. 764 through 765).

The fibre diameter D (μm) is obtained such that fibre cross section of ten of the cut fibres are measured with an S-4000 field-emission scanning electron microscope manufactured by Hitachi, Ltd., and the average is used as the fibre diameter. The fibre diameter D is measured according to <Method for measuring fibre diameter> described above.

Thereafter, the inter-fibre distance of each layer is multiplied by a proportion of the thickness of the layer in the total thickness of the multi-layer structure, and a total of the thus-obtained values of the layers is obtained. Accordingly, an inter-fibre distance of constituent fibres of a target sheet having a multi-layer structure is obtained. For example, in an SMS nonwoven fabric having a triple-layer structure constituted by two S layers and an M layer, the two S layers are used as one layer, and if the total thickness t of the triple-layer structure is 0.11 mm, the thickness t of the S layers is 0.1 mm, the inter-fibre distance LS of the S layers is 47.8 μm, the thickness t of the M layer is 0.01 mm, and the inter-fibre distance LS of the M layer is 3.2 μm, the inter-fibre distance of constituent fibres of the SMS nonwoven fabric is 43.8 μm [= (47.9 × 0.1 + 3.2 × 0.01)/0.11].

[Equation 1]

$$\text{average inter-fiber distance } (\mu m) = 10^6 \sqrt{\frac{t \cdot \rho \cdot \pi D^2}{4 \cdot w \cdot 10^9}} - D \cdots (1)$$

D : fiber diameter (μm)
ρ : fiber resin density (g/cm$^3$)
t : thickness (mm)
w : basis weight (g/m$^2$)

A ratio of the inter-fibre distance of constituent fibres (hydrophilic fibres 14A) of the first hydrophilic layer 11A to the inter-fibre distance of constituent fibres (hydrophobic fibres 15) of the hydrophobic layer 12, that is, a ratio of the inter-fibre distance of constituent fibres (hydrophilic fibres 14A) of the first hydrophilic layer 11A (former) to the inter-fibre distance of constituent fibres (hydrophobic fibres 15) of the hydrophobic layer 12 (latter), that is, the former/the latter, is preferably 0.01 or more and more preferably 0.03 or more, and preferably 0.9 or less and more preferably 0.8 or less, based on the premise that the former < the latter as described above.

The inter-fibre distance of constituent fibres (hydrophilic fibres 14A) of the first hydrophilic layer 11A is preferably 3 μm or more and more preferably 5 μm or more, and preferably 100 μm or less and more preferably 70 μm or less.

The inter-fibre distance of constituent fibres (hydrophobic fibres 15) of the hydrophobic layer 12 is preferably 30 μm or more and more preferably 50 μm or more, and preferably 300 μm or less and more preferably 250 μm or less.

The relationship among inter-fibre distances of constituent fibres of fibre layers applies to the relationship of fibre diameters of the constituent fibres. With respect to the inter-fibre distance, since first hydrophilic layer 11A < hydrophobic layer 12 as described above, similar relationship applies to the fibre diameters. Specifically, a ratio of the fibre diameter of constituent fibres (hydrophilic fibres 14A) of the first hydrophilic layer 11A to the fibre diameter of constituent fibres (hydrophobic fibres 15) of the hydrophobic layer 12, that is, a ratio of the fibre diameter of constituent fibres (hydrophilic fibres 14A) of the first hydrophilic layer 11A (former) to the fibre diameter of constituent fibres (hydrophobic fibres 15) of the hydrophobic layer 12 (latter), that is, the former/the latter, is preferably 0.02 or more and more preferably 0.05 or more, and preferably 0.9 or less and more preferably 0.8 or less, based on the premise that the former < the latter, similarly to the inter-fibre distance.

The fibre diameter of constituent fibres (hydrophilic fibres 14A) of the first hydrophilic layer 11A is preferably 0.5 μm or more and more preferably 1 μm or more, and preferably 30 μm or less and more preferably 20 μm or less.

The fibre diameter of constituent fibres (hydrophobic fibres 15) of the hydrophobic layer 12 is preferably 3 μm or more and more preferably 5 μm or more, and preferably 40 μm or less and more preferably 30 μm or less.

From the same viewpoint as that for setting the inter-fibre distance and the fibre diameter in the ranges described above, that is, from the viewpoint of further ensuring a density gradient of fibres in the laminated structure 13 in which the density increases from the hydrophobic layer 12 (second surface 10b) inward in the thickness direction Z, the first hydrophilic layer 11A preferably has a higher ellipticity (major axis length/minor axis length) of a cross section of constituent fibres than that of the hydrophobic layer 12. In a case where the cross section of constituent fibres is a complete circle, the ellipticity is 1 (one), and as the degree of flatness of the cross section increases (as the shape approaches an ellipse), the ellipticity increases from 1 (one). Examples of a method for obtaining fibres having a large degree of flatness of the cross section, that is, fibres having an ellipticity larger than 1 (one) include (1) a method of spinning fibres with a spinneret having an ellipticity corresponding to a desired ellipticity and (2) a method of performing a postprocess such as pressurization on a stack of fibres obtained using a typical spinneret (having a complete circular or a similar shape in cross section). From the viewpoint of ensuring orientation of the major axis direction of flat fibres in a plane direction of a nonwoven fabric, method (2) is especially preferable in the present invention. Method (2) can be performed by, for example, densifying a fibre aggregate (nonwoven fabric) including fibres having a complete circular shape in cross section by pressurisation while heating the fibre aggregate as necessary, and can be performed by a calendar process described later. The ellipticity of fibres is measured by the following method.

<Method for measuring fibre ellipticity>

In a manner similar to <Method for measuring fibre diameter> described above, a sample piece is prepared, and a cross section thereof is observed with an electron microscope. In a case where a measurement target is a laminated nonwoven fabric having a laminated structure similar to that of the laminated structure 13 described above, for example, a length in the width direction (major axis length) relative to the longitudinal direction of fibres and a length in the thickness direction (minor axis length) of fibres facing the second surface and fibres having fibre diameters smaller than those of the fibres facing the second surface are measured, and the major axis length is divided by the minor axis length, thereby calculating an ellipticity of one fibre. This ellipticity is measured for 10 fibres, and the average is used as an ellipticity.

A ratio of the ellipticity of constituent fibres (hydrophilic fibres 14A) of the first hydrophilic layer 11A to the ellipticity of constituent fibres (hydrophobic fibres 15) of the hydrophobic layer 12, that is, a ratio of the ellipticity of constituent fibres (hydrophilic fibres 14A) of the first hydrophilic layer 11A (former) and the ellipticity of constituent fibres (hydrophobic fibres 15) of the hydrophobic layer 12 (latter), that is, the former/the latter, is preferably 1.02 or more and more preferably 1.09 or more, and preferably 50 or less and more preferably 45 or less, based on the premise that the former > the latter as described above.

The ellipticity of constituent fibres (hydrophilic fibres 14A) of the first hydrophilic layer 11A is preferably 1.2 or more and more preferably 1.25 or more, and preferably 50 or less and more preferably 45 or less.

The ellipticity of constituent fibres (hydrophobic fibres 15) of the hydrophobic layer 12 is preferably 1 or more and more preferably 1.02 or more, and preferably 1.18 or less and more preferably 1.15 or less.

All the constituent fibres (hydrophilic fibres 14A) of the first hydrophilic layer 11A are preferably flat fibres, but are not limited to such fibres. From the viewpoint of providing a large number of portions where the inter-fibre distance is short, the proportion of flat fibres in constituent fibres (hydrophilic fibres 14A) of the first hydrophilic layer 11A obtained from an electron microscopy image is preferably 40% or more and more preferably 50% or more of a unit area.

From the viewpoint of enhancing liquid absorbency, it is preferable that not only the relationship described above is established between the hydrophobic layer 12 and the first hydrophilic layer 11A but also a similar relationship is established between the first hydrophilic layer 11A and the second hydrophilic layer 11B.

That is, a ratio of the fibre diameter of constituent fibres (hydrophilic fibres 14A) of the first hydrophilic layer 11A to the fibre diameter of constituent fibres (hydrophilic fibres 14B) of the second hydrophilic layer 11B, that is, a ratio of the fibre diameter of constituent fibres (hydrophilic fibres 14A) of the first hydrophilic layer 11A (the former) to the fibre diameter of constituent fibres (hydrophilic fibres 14B) of the second hydrophilic layer 11B (the latter) preferably satisfies the former > the latter, and in this case, the ratio of the former to the latter, that is, the former/the latter, is preferably 1.2 or more and more preferably 1.25 or more, and preferably 70 or less and more preferably 50 or less. In this case, the fibre diameter of constituent fibres (hydrophilic fibres 14B) of the second hydrophilic layer 11B is preferably 0.4 $\mu$m or more and more preferably 0.7 $\mu$m or more, and preferably 25 $\mu$m or less and more preferably 18 $\mu$m or less.

A ratio of the inter-fibre distance of constituent fibres (hydrophilic fibres 14A) of the first hydrophilic layer 11A to the inter-fibre distance of constituent fibres (hydrophilic fibres 14B) of the second hydrophilic layer 11B, that is, a ratio of the inter-fibre distance of constituent fibres (hydrophilic fibres 14A) of the first hydrophilic layer 11A (the former) to the inter-fibre distance of constituent fibres (hydrophilic fibres 14B) of the second hydrophilic layer 11B (the latter) preferably satisfies the former > the latter, and in this case, a ratio of the former and the latter, that is, the former/the latter, is preferably 1.1 or more and more preferably 1.2 or more, and preferably 50 or less and more preferably 45 or less. In this case, the inter-fibre distance of constituent fibres (hydrophilic fibres 14B) of the second hydrophilic layer 11B is preferably 2 $\mu$m or more and more preferably 4 $\mu$m or more, and preferably 90 $\mu$m or less and more preferably 65 $\mu$m or less.

A ratio of the ellipticity of constituent fibres (hydrophilic fibres 14A) of the first hydrophilic layer 11A to the ellipticity of constituent fibres (hydrophilic fibres 14B) of the second hydrophilic layer 11B, that is, a ratio of the ellipticity of constituent fibres (hydrophilic fibres 14A) of the first hydrophilic layer 11A (the former) to the ellipticity of constituent fibres (hydrophilic fibres 14B) of the second hydrophilic layer 11B (the latter) preferably satisfies the former < the latter, and in this case, the ratio of the former and the latter, that is, the former/the latter, is preferably 0.02 or more and more preferably 0.05 or more, and preferably 0.9 or less and more preferably 0.85 or less. In this case, the ellipticity of constituent fibres (hydrophilic fibres 14B) of the second hydrophilic layer 11B is preferably 1.25 or more and more preferably 1.3 or more, and preferably 60 or less and more preferably 55 or less.

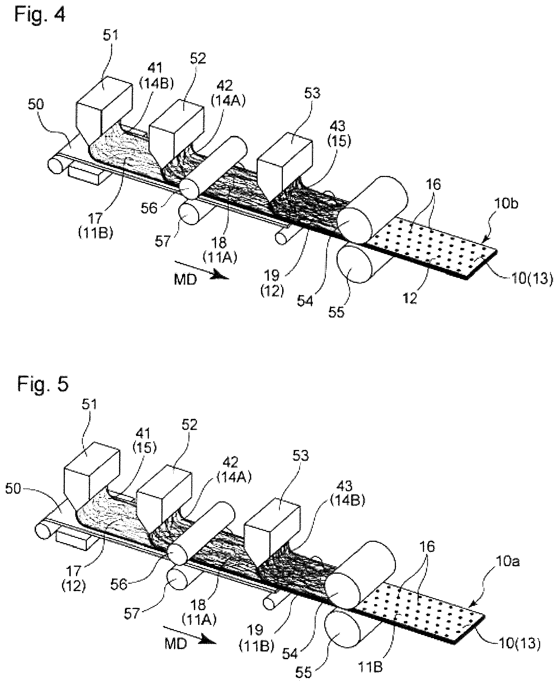

In the laminated nonwoven fabric 10, the interlayer fused portions 16 are formed by pressing a precursor of the laminated structure 13 toward the first surface 10a from the second surface 10b (see Fig. 4, where in Fig. 4, the second laminated body denoted by character 19 corresponds to the "precursor of the laminated structure 13"). This formation method causes the interlayer fused portions 16 to be depressed from the second surface 10b toward the first surface 10a, as illustrated in Fig. 1. In the laminated nonwoven fabric 10, the plurality of interlayer fused portions 16 are dispersed in each of the first surface 10a and the second surface 10b. In the laminated nonwoven fabric 10, at least the second surface 10b is not a flat surface having substantially no concavo-convex shapes (projections and depressions), but is an concavo-convex surface having concavo-convex shapes. In the laminated nonwoven fabric 10, the first surface 10a may be a concavo-convex surface, and both of the first surface 10a and the second surface 10b may be concavo-convex surfaces.

As described above, in a case where one or both of the first surface 10a and the second surface 10b of the laminated nonwoven fabric 10 have concavo-convex shapes, in application of the laminated nonwoven fabric 10 to a constituent that can contact the skin of a user in an absorbent article (e.g., a topsheet or a sweat absorbing sheet), when the laminated nonwoven fabric 10 is disposed such that the concavo-convex surface (e.g., the hydrophobic second surface 10b) contacts the skin of the user, space is formed between the laminated nonwoven fabric 10 and the skin of the user so that damp generated from body fluid such as excreted sweat and urine can be effectively diffused through the space. Thus, surface dryness of the laminated nonwoven fabric 10 can be enhanced, which can lead to enhancement of wearing comfort of the absorbent article.

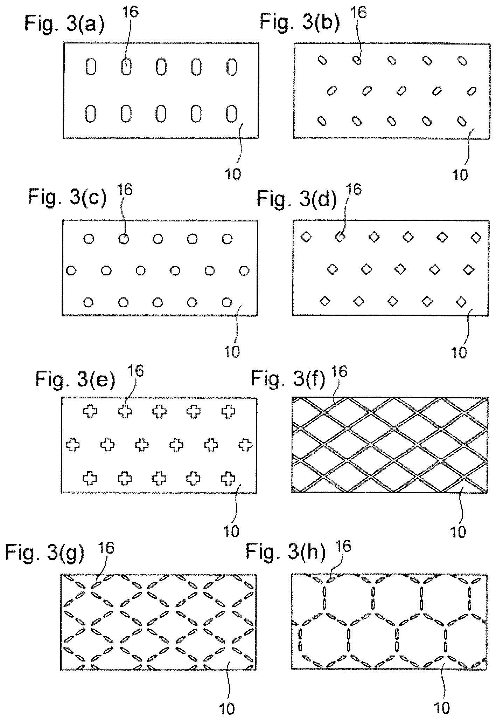

Fig. 3 illustrates an example of a pattern (shape and arrangement in plan view) of the interlayer fused portions 16. In the first surface 10a or the second surface 10b, the pattern of the interlayer fused portions 16 is not limited to the example illustrated in Fig. 3, and a desired pattern may be employed within the range of the gist of the present invention.

Figs. 3(a) through 3(e) illustrate patterns in each of which the interlayer fused portions 16 having a predetermined shape in plan view are dispersed in a surface direction (direction orthogonal to the thickness direction of the laminated nonwoven fabric 10). The shape of the interlayer fused portions 16 in plan view is an oval in Figs. 3(a) and 3(b), is a circle in Fig. 3(c), a rectangle or a rhombus in Fig. 3(d), and is a cross in Fig. 3(e). In Fig. 3(a), major axis directions of the plurality of interlayer fused portions 16 that are oval in plan view coincide with each other, whereas in Fig. 3(b), a plurality of types of interlayer fused portions 16 that are oval in plan view are dispersed with their major axis directions oriented in different directions. Each of Figs. 3(f) through 3(h) illustrates a pattern in which the interlayer fused portions 16 that are linear in plan view are arranged in predetermined directions. In Fig. 3(f), the plurality of continuous linear interlayer fused portions 16 intersect with each other such that the interlayer fused portions 16 form a lattice pattern as a whole. Fig. 3(g) is a variation of the lattice pattern of Fig. 3(f) in which the continuous linear pattern is changed to a discontinuous pattern, specifically, a pattern employing discontinuous lines (intermittent lines) in each of which the relatively short interlayer fused portion 16 is intermittently arranged in a predetermined direction. Fig. 3(h) is another example of the pattern of the discontinuous linear interlayer fused portions 16 in Fig. 3(g), specifically, an example in which the interlayer fused portions 16 are arranged in a honeycomb pattern.

The interlayer fused portions 16 are high-density fused portions in which constituent fibres are arranged at a density higher than those in peripheral portions and are fused together, and in some pressurising and heating conditions in formation, for example, a fibre form of thermoplastic fibres as constituent fibres is lost and is changed to a film form. From the viewpoint of ensuring the advantages described above, however, it is preferable that the fibre form of constituent fibres is kept in the peripheral portions of the interlayer fused portions 16 and their vicinities. The film form of centre portions of the interlayer fused portions 16 advantageously prevents liquid back through the fused portions. The high-density fibre form of constituent fibres in the peripheral portion of the interlayer fused portions 16 and their vicinities advantageously increases a capillary pressure so that a liquid drawing property can be enhanced. The fibre form of constituent fibres in the interlayer fused portions 16 depends mainly on conditions of an embossing in forming the interlayer fused portions 16. If heating and pressurising conditions in the embossing are relatively weak, the fibre form of constituent fibres can be easily maintained.

As described above, since the peripheral portions of the interlayer fused portions 16 and their vicinities serve as liquid drawing portions in absorbing liquid from the second surface 10b, from the viewpoint of enhancing liquid absorbency of the laminated nonwoven fabric 10, the second surface 10b has an interlayer fused portion dispersion region in which the plurality of interlayer fused portions 16 are dispersed in the surface direction. In particular, in a case where the laminated nonwoven fabric 10 is used as a sweat absorbing sheet capable of absorbing sweat, the second surface 10b preferably has the interlayer fused portion dispersion region because of the possibility of further enhancement of sweat absorbing property. The entire region of the second surface 10b may be the interlayer fused portion dispersion region, or only a part of the second surface 10b may be the interlayer fused portion dispersion region. The proportion of the area of the interlayer fused portion dispersion region in the entire area of the second surface 10b is preferably 70% or more and more preferably 80% or more.

With regard to arrangement of the interlayer fused portions 16 in the interlayer fused portion dispersion region, in a case where a virtual circle with a radius of 2 mm is disposed at any position in the interlayer fused portion dispersion region of the second surface 10b, a part or a whole of at least one of the interlayer fused portions 16 is included in the virtual circle. Here, the expression in which "in a case where a virtual circle with a radius of 2 mm is disposed at any position in the interlayer fused portion dispersion region of the second surface 10b, a part or a whole of at least one of the interlayer fused portions 16 is included in the virtual circle" means that "in a case where 10 virtual circles are provided in the interlayer fused portion dispersion region of the second surface 10b, the interlayer fused portions 16 may not be included in one or two of the ten virtual circles at all, and it is sufficient that a part or a whole of the interlayer fused portions 16 is included in at least one of the remaining eight virtual circles." The virtual circles are based on the assumption of sweat excreted portions (sweat glands) dispersed on the skin surface of the human body, and the structure described above enables the laminated nonwoven fabric 10 to absorb sweat more efficiently. In particular, it is more effective that the interlayer fused portions 16 satisfy the condition described above with the radius of the virtual circle being set at 1.5 mm.

The peripheral portions of the interlayer fused portions 16 and their vicinities are liquid drawing portions of the second surface 10b, and thus, to obtain practically sufficient liquid absorbency, a certain number of the portions are necessary, that is, the perimeter of the interlayer fused portions 16 needs to be a certain length or more. In a case where the second surface 10b includes the interlayer fused portions 16 in a number less than or equal to an upper limit described later, this case is preferable because the amount of liquid that can be absorbed and held in the hydrophilic layer 11 increases. From this viewpoint, in a case where the proportion of the total area (total area of the plurality of interlayer fused portions 16 if the second surface 10b has the plurality of interlayer fused portions 16) of the interlayer fused portions 16 to the area of the second surface 10b, that is, the area proportion of the interlayer fused portions 16 is preferably 22% or less and more preferably 20% or less. From the viewpoint of additionally obtaining strength of the laminated nonwoven fabric 10, the lower limit of the proportion is preferably 5% or more and more preferably 6% or more.

The interlayer fused portions 16 are continuous in the entire thickness direction Z of the laminated structure 13 (laminated nonwoven fabric 10) and the pattern (shape and arrangement in plan view) of the interlayer fused portions 16 is substantially the same in the first surface 10a and the second surface 10b. Thus, description of the pattern of the interlayer fused portions 16 of the second surface 10b herein (e.g., the interlayer fused portion dispersion region and the area proportion of the interlayer fused portions 16) is also applicable to the first surface 10a, without otherwise specified.

The basis weight of the laminated nonwoven fabric 10 is not limited to a specific weight, and can be appropriately adjusted depending on application or other factors. For example, in a case where the laminated nonwoven fabric 10 is used as a constituent (e.g., a topsheet or a sweat absorbing sheet) of an absorbent article such as a nappy or a sanitary towel, from the viewpoint of preventing bulkiness while obtaining practically sufficient strength, the basis weight of the laminated nonwoven fabric 10 is preferably 8 $g/m^2$ or more and more preferably 10 $g/m^2$ or more, and preferably 38 $g/m^2$ or less and more preferably 35 $g/m^2$ or less.

In a case where liquid to be absorbed first contacts the hydrophobic layer 12, the hydrophilic layer 11 (the first hydrophilic layer 11A and the second hydrophilic layer 11B) affects a liquid absorbing capacity of the interlayer fused portions 16 and their peripheral portions in the second surface 10b of the hydrophobic layer 12. Typically, as the basis weight of the hydrophilic layer 11 increases, the absorbing capacity increases and the strength increases. From the viewpoint of enhancing wearing comfort in application as a constituent of an absorbent article by reducing the bulk and maintaining softness, the basis weight of the hydrophilic layer 11 is preferably less than or equal to an upper limit described later. In view of the foregoing, the basis weight of the hydrophilic layer 11, that is, the sum of the basis weight of the first hydrophilic layer 11A and the basis weight of the second hydrophilic layer 11B is preferably 5 $g/m^2$ or more and more preferably 7 $g/m^2$ or more, and preferably 25 $g/m^2$ or less and more preferably 20 $g/m^2$ or less. The first hydrophilic layer 11A and the second hydrophilic layer 11B may have the same basis weight or different basis weights.

The hydrophobic layer 12 typically contacts liquid first in the case of using the laminated nonwoven fabric 10 for liquid absorption. In this use form, if the hydrophobic layer 12 has a relatively small basis weight and a relatively small thickness, the hydrophobic layer 12 facilitates liquid absorption through the interlayer fused portions 16 and their peripheries in the second surface 10b. To maintain strength and obtain a sufficient liquid back suppression effect, the basis weight of the hydrophobic layer 12 is preferably less than or equal to a lower limit described later. In view of the foregoing, the basis weight of the hydrophobic layer 12 is preferably 3 $g/m^2$ or more and more preferably 5 $g/m^2$ or more, and preferably 15 $g/m^2$ or less and more preferably 13 $g/m^2$ or less.

The hydrophilic fibres 14A and 14B that are main constituent fibres of the hydrophilic layer 11 (the first hydrophilic layer 11A and the second hydrophilic layer 11B) are typically obtained by performing a hydrophilic treatment on inherently hydrophobic thermoplastic fibres. That is, the hydrophilic layer 11 is an aggregate of hydrophobic fibres (e.g., hydrophobic fibres 15 that are thermoplastic fibres usable as constituent fibres of the hydrophobic layer 12) subjected to a hydrophilic treatment. As described above, this hydrophilic treatment is typically applicable to a plasma treatment as well as application of a hydrophilising agent to fibres or a fibre aggregate and kneading of a hydrophilising agent into fibres, and a treatment using hydrophilising agent is typically employed.

An example of the first hydrophilic layer 11A and the second hydrophilic layer 11B is a hydrophilic nonwoven fabric including fibres in which a hydrophilising agent is kneaded. In each of the hydrophilic layers 11A and 11B in this form, the hydrophilising agent is not attached to the surfaces of the hydrophilic fibres 14A and 14B as main constituent fibres, but is included in the hydrophilic fibres 14A and 14B. Each of the hydrophilic layers 11A and 11B in this form does not have a hydrophilic degree gradient in the thickness direction Z and has a uniform hydrophilic degree, based on the assumption that constituent fibres are uniformly distributed in the entire layer.

The laminated structure in the laminated nonwoven fabric according to the present invention may include another layer, in addition to the hydrophilic layers (the first hydrophilic layer and the second hydrophilic layer) including hydrophilic fibres and the hydrophobic layer including hydrophobic fibres. Specifically, the laminated structure includes elastic fibres, for example.

The elastic fibres may be interposed between any layers in the laminated structure 13. In this case, the elastic fibres are typically in an aggregate form, that is, serve as an elastic fibre layer and are interposed between layers in the laminated structure 13. The interposition of elastic fibres (elastic fibre layers) between layers in the laminated structure provides the laminated nonwoven fabric with elasticity so that the laminated nonwoven fabric can be applied in a wider range. For example, in the laminated nonwoven fabric 10 (laminated structure 13) illustrated in Fig. 1, elastic fibres (elastic fibre layer) are interposed between the first hydrophilic layer 11A and the second hydrophilic layer 11B or between the first hydrophilic layer 11A and the hydrophobic layer 12. In this case, the laminated nonwoven fabric 10 (laminated structure 13) is elastic in the same direction as an elastic direction of the elastic fibres (elastic fibre layer).

The elastic fibres (elastic fibre layer) can be extended, and preferably has a property (elasticity) in which when a force is released from a state where the elastic fibres are extended 1.3 times as long as the original length thereof (the elastic fibres have a length 1.3 times as large as the original length), the elastic fibres return to a length less than or equal to 1.1 times as large as the original length. An inelastic member (inelastic fibres) is a member not having such "elasticity," that is, a member having a property in which when a force is released from a state where the member is extended 1.3 times as long as the original length (the member has a length 1.3 times as large as the original length), the member does not return to a length less than or equal to 1.1 times as large as the original length.

As a material for the elastic fibres, a resin material such as natural rubber or thermoplastic elastomer can be used. Examples of the resin material for thermoplastic elastomer include: styrene-based elastomers such as styrene-butadiene-styrene (SBS), styrene-isoprene-styrene (SIS), styrene-ethylene-butadiene-styrene (SEBS), and styrene-ethylene-propylene-styrene (SEPS); olefin-based elastomers (ethylene-based $\alpha$-olefin elastomer, and propylene-based elastomer obtained by copolymerizing ethylene, butene, and octene, for example); a polyester-based elastomer; and a polyurethane-based elastomer.

Next, a method for manufacturing a laminated nonwoven fabric according to the present invention will be described with reference to the drawings based on a preferred embodiment. Figs. 4 and 5 schematically illustrate examples of a method for manufacturing the laminated nonwoven fabric 10 described above, as embodiments of the method for manufacturing a laminated nonwoven fabric according to the present invention. As described above, the laminated nonwoven fabric 10 has the laminated structure 13 of the fibre layer (nonwoven fabric) including the hydrophilic fibres 14A and 14B and the hydrophobic fibres 15 as long fibres (thermoplastic fibre), and layers constituting the laminated structure 13 are fused to one another in the interlayer fused portions 16.

The method for manufacturing the laminated nonwoven fabric 10 illustrated in Fig. 4 includes: the step of spinning a resin to obtain first fibres 41 and depositing the first fibres 41 to form a base web 17 (base web forming step); the step of spinning a resin to obtain second fibres 42 and depositing the second fibres 42 on the base web 17 to form a first laminated body 18 (first lamination step); the step of spinning a resin to obtain third fibres 43 and depositing the third fibres 43 on the first laminated body 18 to from a second laminated body 19 (second lamination step); the step of heating the second laminated body 19 while partially compressing the second laminated body 19 in a thickness direction to form interlayer fused portions 16 (interlayer fusing step).

In the method for manufacturing a laminated nonwoven fabric according to the present invention, one of the first fibres 41 (fibres spun for the first time) or the third fibres 43 (fibres spun for the third time) include the hydrophobic fibres 15, the other fibres include the hydrophilic fibres 14B, and the second fibres 42 (fibres spun for the second time) include the hydrophilic fibres 14A having a fibre diameter smaller than that of the hydrophobic fibres 15. That is, in the method for manufacturing a laminated nonwoven fabric according to the present invention, any one of hydrophilic fibres or hydrophobic fibres may be spun for the first time and the third time in the three spinning steps, but hydrophilic fibres having a fibre diameter smaller than that of the hydrophobic fibres are always spun for the second time.

In the method for manufacturing the laminated nonwoven fabric 10 illustrated in Fig. 4, the first fibres 41 include the hydrophilic fibres 14B, and the third fibres 43 include the hydrophobic fibres 15. On the other hand, in a method for manufacturing the laminated nonwoven fabric 10 illustrated in Fig. 5 described later, the first fibres 41 include the hydrophobic fibres 15, and the third fibres 43 include the hydrophilic fibres 14B.

In the method for manufacturing the laminated nonwoven fabric 10 illustrated in Fig. 4, first, the first fibres 41 (hydrophilic fibres 14B) are spun out from a spinneret of a first spinning head 51 disposed above a conveyor 50, and are deposited on the conveyor 50 in a web form, thereby forming the base web 17. Thereafter, the resulting base web 17 is conveyed by the conveyor 50 in one direction indicated by characters MD, and in the middle of the conveyance, the second fibres 42 (hydrophilic fibres 14A) are spun out from a spinneret of the second spinning head 52 disposed above the conveyor 50 and are deposited directly on the base web 17, thereby forming the first laminated body 18. Subsequently, the first laminated body 18 is conveyed by the conveyor 50, and in the middle of the conveyance, the third fibres 43 (hydrophobic fibres 15) are spun out from a spinneret of the third spinning head 53 disposed above the conveyor 50 and are deposited directly on the first laminated body 18, thereby forming the second laminated body 19. Then, the second laminated body 19 is conveyed to be supplied between a pair of embossing rollers 54 and 55, and is subjected to heat embossing, thereby forming interlayer fused portions 16 (interlayer fusing step). The embossing roller 54 is disposed above the second laminated body 19 that is being conveyed, and directly contacts a deposit (web) of the spun third fibres 43 (hydrophobic fibres 15) during the heat embossing. The embossing roller 55 is disposed below the second laminated body 19 that is being conveyed, and contacts a deposit (web) of the first fibres 41 (hydrophilic fibres 14B) during the heat embossing. With respect to the outer peripheral surfaces of the rollers 54 and 55 that contact the second laminated body 19 as a processing target, the outer peripheral surface of the embossing roller 55 does not have concavo-convex shapes (projections and depressions), whereas the outer peripheral surface of the embossing roller 54 has projections (not shown) in a pattern corresponding to a pattern of the interlayer fused portions 16 in the laminated nonwoven fabric 10 as a manufacturing object. In nip portions of the rollers 54 and 55 having such a structure, the second laminated body 19 is compressed in the thickness direction while being heated to a temperature greater than or equal to a melting point of thermoplastic fibres (the hydrophilic fibres 14A and 14B and the hydrophobic fibres 15) included in the second laminated body 19. Accordingly, the second laminated body 19 is partially compressed by the projections of the embossing roller 54 from the deposit of the third fibres 43 (hydrophobic fibres 15) toward the deposit of the first fibres 41 (hydrophilic fibres 14B), and the interlayer fused portions 16 are formed in the compressed portions. This interlayer fusing step changes the second laminated body 19 into a nonwoven fabric, and the layers are united through the interlayer fused portions 16. In the manner described above, there obtained the laminated nonwoven fabric 10 having the laminated structure 13 constituted by the second hydrophilic layer 11B of the base web 17, the first hydrophilic layer 11A of the deposit (web) of the second fibres 42 (hydrophilic fibres 14A), and the hydrophobic layer 12 of the deposit (web) of the third fibres 43 (hydrophobic fibres 15). In the thus-obtained laminated nonwoven fabric 10, the interlayer fused portions 16 are depressed from the second surface 10b toward the first surface 10a, as illustrated in Fig. 1.

The method for manufacturing the laminated nonwoven fabric 10 illustrated in Fig. 5 is the same as the method for manufacturing the laminated nonwoven fabric 10 illustrated in Fig. 4, except for a change of the order of spinning of constituent fibres of the laminated nonwoven fabric 10. Specifically, in the method for manufacturing the laminated nonwoven fabric 10 illustrated in Fig. 4, the hydrophilic fibres 14B (first fibres 41), the hydrophilic fibres 14A (second fibres 42), and the hydrophobic fibres 15 (third fibres 43) are spun in this order, whereas in the method for manufacturing the laminated nonwoven fabric 10 illustrated in Fig. 5, the hydrophobic fibres 15 (first fibres 41), the hydrophilic fibres 14A (second fibres 42), and the hydrophilic fibres 14B (third fibres 43) are spun in this order. With this difference in the spinning order, in the laminated nonwoven fabric 10 obtained by the method for manufacturing the laminated nonwoven fabric 10 illustrated in Fig. 5, the interlayer fused portions 16 are depressed from the first surface 10a toward the second surface 10b, in a manner opposite to the laminated nonwoven fabric 10 obtained by the method for manufacturing the laminated nonwoven fabric 10 illustrated in Fig. 4.

In the methods for manufacturing the laminated nonwoven fabric 10 illustrated in Figs. 4 and 5, a process of heating an object while compressing the object in the thickness direction, such as heat embossing, is not performed on any of the base web 17, the first laminated body 18, and the second laminated body 19, before the interlayer fusing step.

In each of the laminated nonwoven fabrics 10 as manufactured products of the manufacturing methods illustrated in Figs. 4 and 5, the first surface 10a is formed of the deposit of one of the first fibres 41 or the third fibres 43 including the hydrophilic fibres 14B, and the second surface 10b of the laminated nonwoven fabric 10 is formed of the deposit of one of the first fibres 41 or the third fibres 43 including the hydrophobic fibres 15.

Each of the laminated nonwoven fabrics 10 fabricated by the manufacturing methods illustrated in Figs. 4 and 5 includes the first surface 10a and the second surface 10b located opposite to the first surface 10a, and the first surface 10a is formed of the deposit of one of the first fibres 41 or the third fibres 43 including the hydrophilic fibres 14B, and the second surface 10b is formed of the deposit of one of the first fibres 41 or the third fibres 43 including the hydrophobic fibres 15.

The hydrophilic fibres 14A and 14B used in the manufacturing methods of the laminated nonwoven fabric 10 illustrated in Figs. 4 and 5 are obtained by spinning a resin mixed with a hydrophilising agent. Thus, in the laminated nonwoven fabrics 10 as manufactured products of the manufacturing methods, the first hydrophilic layer 11A including the hydrophilic fibres 14A and the second hydrophilic layer 11B including the hydrophilic fibres 14B are hydrophilic nonwoven fabrics including fibres in which the hydrophilising agent is kneaded. In the hydrophilic layers 11A and 11B in this form, on the premise that the constituent fibres are uniformly distributed in the entire each layer as described above, the hydrophilic layers 11A and 11B do not have a hydrophilic degree gradient in the thickness direction Z, and the hydrophilic degree is uniform.

As described above, in the case of obtaining the hydrophilic fibres 14A and 14B by spinning a resin mixed with the hydrophilising agents, that is, in the case of using the hydrophilic fibres 14A and 14B in which the hydrophilising agents are kneaded, the contents of the hydrophilising agents may be different between the fibres 14A and 14B. Specifically, in the manufacturing methods illustrated in Figs. 4 and 5, for example, the content of the hydrophilising agent in the hydrophilic fibres 14A as the second fibres 42 is reduced to be smaller than that of one of the first fibres 41 or the third fibres 43 including the hydrophilic fibres 14B. Accordingly, in the hydrophilic layer 11 of the laminated nonwoven fabric 10 as a manufactured product, the first hydrophilic layer 11A has a relatively small content of the hydrophilising agent, and the second hydrophilic layer 11B has a relatively large content of the hydrophilising agent. Consequently, the hydrophilic layer 11 has a hydrophilic degree gradient in which the first hydrophilic layer 11A has a relatively low hydrophilic degree and the second hydrophilic layer 11B has a relatively high hydrophilic degree. Thus, the laminated structure 13 of the laminated nonwoven fabric 10 as a manufactured product has a hydrophilic degree gradient in which the hydrophilic degree increases stepwise (contact angle measured by the method described above decreases stepwise) from the hydrophobic layer 12 (second surface 10b) that first contacts liquid to be absorbed inward in the thickness direction Z of the laminated structure 13. Thus, liquid absorbency is expected to be further enhanced.

A main feature of this manufacturing method is that all the layers constituting the laminated structure 13 are formed by forming and depositing long fibres by, for example, melt spinning on a manufacturing line of the laminated nonwoven fabric 10 as a manufacturing object. This manufacturing method using such a so-called direct spinning technique is different from a method in which long fibres obtained by melt spinning are temporarily wound by a winder, and then, fibres obtained by drawing or cutting are deposited on a nonwoven fabric fabricated in a different process.

In producing a laminated nonwoven fabric such as the laminated nonwoven fabric 10, the use of a direct spinning technique, as in this manufacturing method, can reduce the base weight of the laminated nonwoven fabric, as compared to a method in which a plurality of nonwoven fabrics are stacked and combined by heat embossing. In particular, layers formed by the direct spinning technique, especially layers of the second fibres 42 and the third fibres 43, can be easily reduced in thickness. In this manufacturing method, the second fibres 42 and the third fibres 43 obtained by spinning are deposited directly on the base web 17 (layer of the first fibres 41) to form the second laminated body 19. Thus, the hydrophilic fibres 14A as the second fibres 42 disposed between the layer of the first fibres 41 and the layer of the third fibres 43 can easily enter layers of the fibres 41 and 43 so that adhesion between these layers is increased. Thus, as described above, in a case where peripheral portions of the interlayer fused portions 16 and their vicinities in the second surface 10b are used as liquid drawing portions, the high-quality laminated nonwoven fabric 10 having high liquid absorbency and high strength can be obtained.

In this manufacturing method, after deposition of the hydrophilic fibres 14A and 14B, a calendar process may be performed on the deposits of these fibres. As widely known, the calendar process is a process in which a fibre aggregate such as a nonwoven fabric is subjected to heat and pressure with calendar rollers so that the density of the fibre aggregate is increased. In the manufacturing methods illustrated in Figs. 4 and 5, the hydrophilic fibres 14A as the second fibres 42 are spun out from the second spinning head 52 and are deposited directly on the base web 17, thereby forming the first laminated body 18. Immediately after the formation of the first laminated body 18, the first laminated body 18 is supplied between a pair of calendar rollers 56 and 57, thereby performing a calendar process on the first laminated body 18 including the deposit (web) of the hydrophilic fibres 14A. The calendar process may be performed on the second laminated body 19 including the deposit (web) of the hydrophilic fibres 14B, or may be performed on the laminated bodies 18 and 19. In the manufacturing method illustrated in Fig. 4, a calendar process may be performed on the deposit (base web 17) of the hydrophilic fibres 14B (first fibres 41).

In this manner, the calendar process performed on the deposit of spun hydrophilic fibres can reduce the inter-fibre distance of the hydrophilic fibres, increase the capillary pressure, and further promote the above-described advantage by the direct spinning technique: "entering of the hydrophilic fibres 14A (constituent fibres of the first hydrophilic layer 11A) into the layer of the base web 17 (the second hydrophilic layer 11B or the hydrophobic layer 12) and the layer of the third fibres 43 (the hydrophobic layer 12 or the second hydrophilic layer 11B)." Thus, properties of the laminated nonwoven fabric 10, such as liquid absorbency and strength, can be further enhanced. The number and arrangement of calendar rollers used for the calendar process are not limited to the illustrated example. For example, a serial type or a tilt type constituted by three calendar rollers, a serial type, an inverted L type, a Z type, or a tilted Z type constituted by four calendar rollers, a Z type or an L type constituted by five calendar rollers may be employed. The temperature in the calendar process is preferably less than or equal to a softening point of constituent fibres of the laminated bodies 18 and 19 as processing targets. The calendar process performed at the softening point or less softens the laminated nonwoven fabric 10 as a manufactured product and comfort to the skin can be enhanced.

In the methods for manufacturing the laminated nonwoven fabric 10 illustrated in Figs. 4 and 5, the first fibres 41, the second fibres 42, and the third fibres 43 are deposited on the upper surface of the conveyor 50, and the deposits (webs) are conveyed in one direction indicated by characters MD A material for the upper surface (contact portions with the webs) of the conveyor 50 serving as a member on which the webs are deposited and which conveys the webs is selected from, for example, fluororesins, polyester resins, and metals. From the viewpoint of enhancing separation of the web from the conveyor 50, fluororesins are preferably selected. The shape of the upper surface of the conveyor 50 can be selected from, for example, a net shape including a plurality of intersecting wire materials and through holes defined by the wire materials, a rubber belt shape including no such through holes. From the viewpoint of preventing degradation of separation due to attachment of a high-temperature web to the upper surface of the conveyor 50 in separation of the web from the conveyor 50, the net shape having high air permeability is preferably selected. In addition, in the case of performing the calendar process described above on the web, if the upper surface of the conveyor 50 has the net shape including wire materials and through holes, portions of the web corresponding to the wire materials are compressed in the thickness direction more strongly than portions corresponding to the through holes and densified. That is, in the case where the upper surface (contact portions with the web or fibres) of the conveyor has the net shape, as the area of the through holes decreases (as the area of the wire materials increases) portions of the web compressed relatively strongly increases, resulting in a more densified laminated nonwoven fabric.

As described above, the laminated nonwoven fabric according to the present invention includes a form in which elastic fibres (elastic fibre layer) are interposed between the first hydrophilic layer 11A and the second hydrophilic layer 11B or between the first hydrophilic layer 11A and the hydrophobic layer 12. In fabricating a laminated nonwoven fabric having such a configuration in which elastic fibres are interposed between layers in the laminated structure, it may be sufficient to employ the step of depositing elastic fibres obtained by spinning a resin directly on the base web 17 or the first laminated body 18, between the base web forming step and the first lamination step or between the first lamination step and the second lamination step.

The following description will be directed to an absorbent article according to the present invention with reference to the drawings based on a preferred embodiment. The absorbent article according to the present invention is characterised by including the laminated nonwoven fabric according to the present invention described above. Specifically, as described above, the laminated nonwoven fabric according to the present invention has the laminated structure of fibre layers (nonwoven fabric) including long fibres (thermoplastic fibres), the laminated structure includes the first surface that is a surface of the laminated nonwoven fabric and the second surface that is the other surface of the laminated nonwoven fabric, the second surface is constituted by the hydrophobic layer including hydrophobic fibres, the hydrophilic layer including hydrophilic fibres is disposed at the first surface side of the hydrophobic layer, and the hydrophilic layer is configured to include the first hydrophilic layer and the second hydrophilic layer arranged in this order from the hydrophobic layer. In the article according to the present invention, the laminated nonwoven fabric is disposed with the second surface facing the skin of a user.

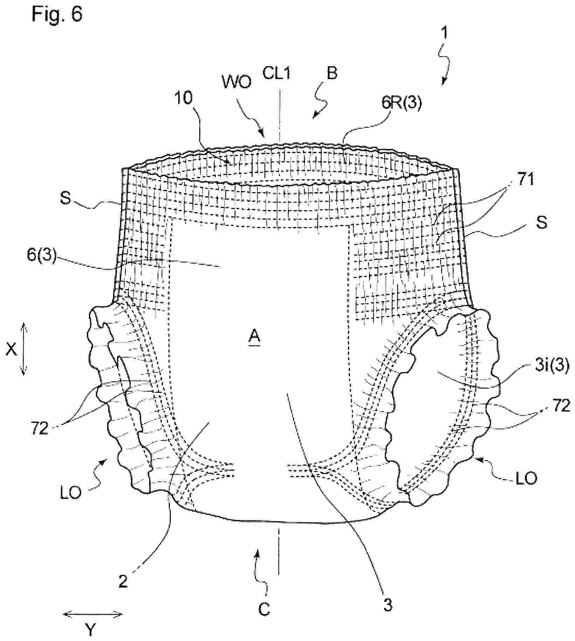

Figs. 6 through 8 illustrate a pull-on nappy 1 that is one embodiment of the absorbent article according to the present invention. Description of the nappy 1 will be mainly directed to components different from those of the laminated nonwoven fabric 10 that is an embodiment of the laminated nonwoven fabric described above, and similar components are denoted by the same characters, and description thereof will not be repeated. Description for the laminated nonwoven fabric 10 will be appropriately applied to components not particularly described.

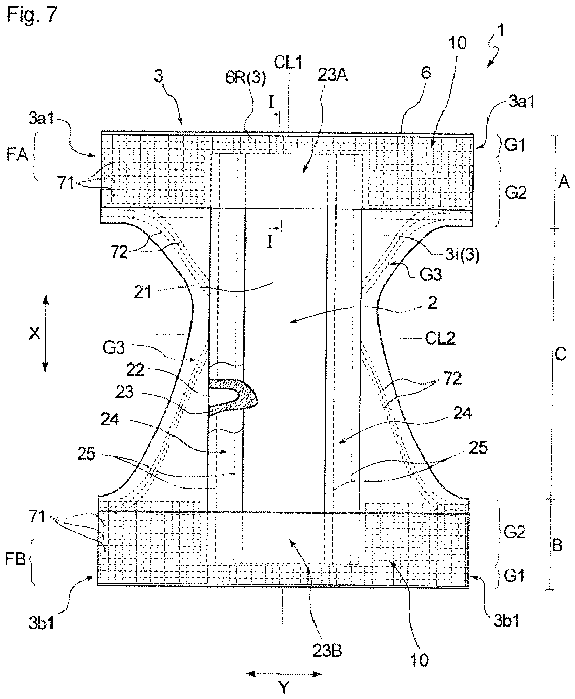

As illustrated in Figs. 6 and 7, the nappy 1 includes a front region A and a rear region B arranged in a longitudinal direction X and a crotch region C disposed between the regions A and B. The nappy 1 also includes an absorbent member 23 extending across the front region A and the rear region B, and a front waist flap FA and a rear waist flap FB located outside respectively from front and rear end portions 23A and 23B of the absorbent member 23 in the longitudinal direction X and extending in a lateral direction Y. Here, the front region A is a region disposed at a front side of the user when the user wears an absorbent article such as a nappy, and the rear region B is a region disposed at a rear side of the user when the user wears the absorbent article such as a nappy. The front region A and the rear region B are waist portions corresponding to the waist of the user while the user wears the nappy 1.

As illustrated in Fig. 7, the front waist flap FA refers to a region that is a combination of a region located outside, in the longitudinal direction X, from an edge of the front end portion 23A of the absorbent member 23 at the front region A side in the longitudinal direction X and extending in the lateral direction Y and a region extending in the lateral direction Y from the front end portion 23A of the absorbent member 23 at the front region A side in the longitudinal direction X. The rear waist flap FB refers to a region that is a combination of a region located outside, in the longitudinal direction X, from an edge of the rear end portion 23B of the absorbent member 23 at the rear region B side in the longitudinal direction X and extending in the lateral direction Y and a region extending in the lateral direction Y from the rear end portion 23B of the absorbent member 23 at the rear region B side in the longitudinal direction X.

As illustrated in Figs. 6 and 7, the nappy 1 includes an absorbent assembly 2 and an outer cover 3 disposed at a non-skin-facing surface side of the absorbent assembly 2 and fixes the absorbent assembly 2. The outer cover 3 includes the front waist flap FA and the rear waist flap FB located outside respectively from the front and rear end portions 23A and 23B in the longitudinal direction X of the absorbent member 23 constituting the absorbent assembly 2 and extending in the lateral direction Y. In the nappy 1, an elasticised region (waist elasticised portion G1 and waist lower elasticised portion G2) are formed in the front waist flap FA and the rear waist flap FB.

As illustrated in Fig. 7, the nappy 1 is a so-called pull-on nappy in which both lateral side edge portions 3a1 and 3a1 of the front region A of the outer cover 3 and both lateral side edge portions 3b1 and 3b1 of the rear region B of the outer cover 3 are joined to form a pair of side seals S and S, a waist opening WO, and a pair of leg openings LO and LO (see Fig. 6). Preferably, when the outer cover 3 of the nappy 1 in its unfolded and stretched state is seen in a plan view as illustrated in Fig. 7, the outer cover 3 is divided into the front region A disposed at the front side of a user while the user wears the nappy 1, the rear region B disposed at the rear side of the user while the user wears the nappy 1, and the crotch region C disposed between the front region A and the rear region B. The unfolded and stretched state of the nappy 1 here refers to a state where the side seals S are torn off so that the nappy 1 is developed, as illustrated in Fig. 7, and the developed nappy 1 are expanded to design dimensions (corresponding to dimensions when the nappy 1 is expanded in a plane under no influence of elastic members) by extending elastic members of parts of the nappy 1.

The "skin-facing surface" herein refers to a surface of the nappy 1 or its constituent (e.g., absorbent assembly 2) facing the skin of the user while the user wears the nappy 1. The "non-skin-facing surface" refers to a surface of the nappy 1 or its constituent toward the side (cloth side) opposite to the skin side of the user while the user wears the nappy 1. In the nappy 1, the longitudinal direction X refers to a direction corresponding to a direction from the front side of the user toward the rear side through the crotch portion, and a direction across the front region A and the rear region B in a state where the nappy 1 is developed in plan and extended. The lateral direction Y refers to a direction orthogonal to the longitudinal direction X, and a width direction of the nappy 1 developed in plan and extended. The nappy 1 is symmetric with respect to a vertical centre line CL1 extending in the longitudinal direction X illustrated in Fig. 7. In Fig. 7, CL2 represents a lateral centre line extending in the lateral direction Y to divide the nappy 1 into two, and orthogonal to the vertical centre line CL1.

In the nappy 1, as illustrated in Fig. 7, when the nappy 1 is in its unfolded and streatched state, the absorbent assembly 2 has an elongated shape that is relatively long in the longitudinal direction X. The absorbent assembly 2 includes a liquid-permeable topsheet 21 forming a skin-facing surface, a sparingly liquid permeable (including water-repellent) backsheet 22 forming a non-skin-facing surface, and a liquid-retentive absorbent member 23 interposed between these sheets 21 and 22. As illustrated in Fig. 7, side portions of the absorbent assembly 2 in the longitudinal direction X (longitudinal direction) are provided with leak-proof cuffs 24 and 24 including an elastic member and disposed in the longitudinal direction X while being extended. Specifically, the leak-proof cuffs 24 are made of a sparingly liquid permeable or water-repellent air-permeable material, and the leak-proof cuff elastic member 25 extended in the longitudinal direction X is disposed near free ends of the leak-proof cuffs 24. In wearing the nappy, contraction of the leak-proof cuff elastic member 25 causes the free end portions of the leak-proof cuffs 24 to stand so that an outflow of body fluid in the lateral direction Y is inhibited.

As illustrated in Fig. 7, the absorbent assembly 2 having the configuration described above is joined, with an assembly fixing adhesive, to a centre portion of the outer cover 3 with the longitudinal direction thereof being matched with the longitudinal direction X of the nappy 1 in its unfolded and stretched state. In this manner, the outer cover 3 is disposed at the non-skin-facing surface side of the backsheet 22 constituting the absorbent assembly 2 in the thickness direction of the nappy 1, and is fixed and joined to the non-skin-facing surface side. Thus, in the nappy 1, the absorbent member 23 constituting the absorbent assembly 2 is disposed across the front region A and the rear region B.

In the nappy 1, as illustrated in Fig. 7, the outer cover 3 includes the front waist flap FA and the rear waist flap FB located outside respectively from the front and rear end portions 23A and 23B in the longitudinal direction X of the absorbent member 23 and extending in the lateral direction Y. As illustrated in Figs. 6 through 8, the outer cover 3 includes an outer sheet 6 at a non-skin-facing surface side forming an outer surface of the nappy 1, and an inner layer sheet 3i disposed at the skin-facing surface side of the outer sheet 6. The outer sheet 6 includes a folded portion 6R in which an extension portion extending from each of front and rear end portions in the longitudinal direction X of the inner layer sheet 3i is folded to the skin-facing surface side of the inner layer sheet 3i. As illustrated in Fig. 7, the folded portion 6R of the outer sheet 6 has a rectangular shape elongated in the lateral direction Y. Each of the front and rear end portions 23A and 23B of the absorbent assembly 2 is covered with the folded portion 6R.

As described above, the nappy 1 includes the front region A and the rear region B that are waist portions corresponding to the waist of the user while the user wears the nappy 1, and as illustrated in Fig. 7, the regions A and B include at least the waist elasticised portion G1 and the waist lower elasticised portion G2 as elasticised regions, and can also include the leg elasticised portions G3. That is, the outer cover 3 includes, in the regions A and B, a plurality of elastic members 71 intermittently disposed in the longitudinal direction X while extending in the lateral direction Y between the outer sheet 6 and the inner layer sheet 3i constituting the outer cover 3 so that the waist elasticised portion G1 and the waist lower elasticised portion G2 are formed as elasticised regions that are elastic in the lateral direction Y in the regions A and B. The outer cover 3 includes a plurality of leg elastic members 72 extending in a predetermined direction from the regions A and B across the crotch region C between the outer sheet 6 and the inner layer sheet 3i, and thus can also include the leg elasticised portion G3 as an elasticised region in the regions A, B, and C.

In the nappy 1, as illustrated in Figs. 6 and 7, the waist elasticised portion G1 is formed in each end flap located outside, in the longitudinal direction X, (at the side opposite to the lateral centre line CL2) from the edges of the front and rear end portions 23A and 23B in the longitudinal direction X of the absorbent member 23 constituting the absorbent assembly 2, in the longitudinal direction X. In the nappy 1, the waist lower elasticised portion G2 is formed in each side flap located between the lower end of the waist elasticised portion G1 toward the lateral centre line CL2 and the lower end of the side seal S, in the longitudinal direction X. The rear waist flap FB and the front waist flap FA are also a region as a combination of the end flap (waist elasticised portion G1) and a part of the side flap (waist lower elasticised portion G2). In the nappy 1, as illustrated in Figs. 6 and 7, the leg elasticised portion G3 is formed at the periphery of the leg opening LO.

As illustrated in Figs. 6 through 8, the nappy 1 includes a sweat absorbing sheet 10 (see Fig. 1) capable of absorbing sweat. The sweat absorbing sheet 10 is the laminated nonwoven fabric 10 as an embodiment of the laminated nonwoven fabric according to the present invention described above. The sweat absorbing sheet 10 is disposed with the second surface 10b (outer surface of the hydrophobic layer 12) facing the skin of the user.

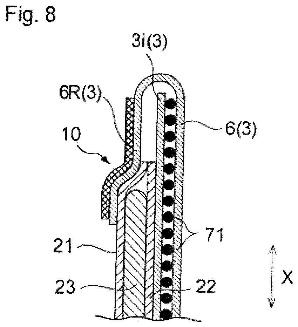

In the nappy 1, the sweat absorbing sheet 10 is used mainly for absorbing sweat around the waist of the user, and is disposed in the elasticised region of the waist portion described above. That is, the sweat absorbing sheet 10 has a shape elongated in one direction in the plan view illustrated in Fig. 7, specifically, a rectangular shape (band shape). The sweat absorbing sheet 10 is disposed across the entire lengths of the front region A and the rear region B as waist portions in the lateral directions Y thereof with the longitudinal direction of the rectangular shape coinciding with the lateral directions Y, and extends at least across the waist elasticised portion G1 and the waist lower elasticised portion G2. Specifically, the sweat absorbing sheet 10 is joined to the skin-facing surface of the folded portion 6R of the outer sheet 6 with a known joining means such as an adhesive, heat sealing, or ultrasonic sealing. The folded portion 6R is disposed at a position closer to the skin of the user than the absorbent member 23 while the user wears the nappy 1 (see Fig. 8). Thus, the sweat absorbing sheet 10 at the skin-facing surface of the folded portion 6R is disposed at a position closer to the skin of the user than the absorbent member 23 while the user wears the nappy 1, and can contact the skin of the user.

In the nappy 1 having a configuration as described above, the sweat absorbing sheet 10 (laminated nonwoven fabric 10) having a sweat absorbing function is disposed closest to the skin of the user of the nappy 1 at the skin-facing surface side of each of the front region A and the rear region B as waist portions, and the sweat absorbing sheet 10 is disposed in the elasticised region that includes the waist elasticised portion G1 and the waist lower elasticised portion G2 and is elastic in the lateral direction Y. Thus, while the user wears the nappy 1, the hydrophobic second surface 10b of the sweat absorbing sheet 10 (see Fig. 1) well fits with the skin of the user by a fastening force due to a contraction force of the elasticised region so that sweat from the skin can be quickly absorbed and exhaled.

Since the sweat absorbing sheet 10 (laminated nonwoven fabric 10) is disposed in the elasticised region in the nappy 1 as described above, from the viewpoint of enhancing fitness for the skin of the user, the sweat absorbing sheet 10 is also preferably elastic in the same direction as the elasticised region, that is, in the lateral direction Y. From this viewpoint, elastic fibres (elastic fibre layer) elastic in the lateral direction Y are preferably disposed between the first hydrophilic layer 11A and the second hydrophilic layer 11B or between the first hydrophilic layer 11A and the hydrophobic layer 12, in the sweat absorbing sheet 10. These elastic fibres (elastic fibre layer) are already described above.

With respect to materials for portions of the nappy 1, the topsheet 21, the backsheet 22, the absorbent member 23, and the leak-proof cuffs 24, for example, constituting the absorbent assembly 2 can be made of various materials typically used for an absorbent article such as a nappy without any particular limitation. For example, as the topsheet 21, a single-layer or multi-layer nonwoven fabric or a perforated film, for example, may be used. As the backsheet 22, a moisture-permeable resin film, for example, may be used. As the absorbent member 23, an absorbent core constituted by absorbent polymer particles and a fibre material covered with tissues may be used. As the leak-proof cuffs 24, a water-repellent single-layer or a multi-layer nonwoven fabric, for example, may be used. Examples of the elastic member (e.g., the leak-proof cuff elastic member 25, the elastic members 71, and the leg elastic members 72) include synthetic rubbers such as styrene-butadiene, butadiene, isoprene, and Neoprene, natural rubber, EVA, flexible polyolefin, and polyurethane. The form of the elastic member is preferably a filamentous form (e.g., rubber threads) having a rectangular, square, circle, oval, or polygonal, for example, cross section, or a cord-like form (e.g., flat rubber), for example. As a means for joining constituents of the nappy 1, various adhesives such as a hot-melt adhesive typically used for absorbent articles of this type may be used.

The present invention has been described based on an embodiment, but the present invention may be appropriately changed without any limitation of the embodiment.

For example, in the nappy 1, the laminated nonwoven fabric 10 as an embodiment of a laminated nonwoven fabric of the present invention is used as the sweat absorbing sheet 10. It is sufficient, however, that the absorbent article of the present invention includes the laminated nonwoven fabric of the present invention and the second surface (hydrophobic surface including interlayer fused portions) of the laminated nonwoven fabric is disposed to face the skin of the user. The laminated nonwoven fabric of the present invention can be used as another constituent except the sweat absorbing sheet, and even in the case of using the laminated nonwoven fabric as the sweat absorbing sheet, the position of the laminated nonwoven fabric is not limited to the position of the sweat absorbing sheet 10 in the nappy 1.

Examples of other application of the laminated nonwoven fabric of the present invention as a constituent of an absorbent article include a topsheet disposed at a position closer to the skin of a user than an absorbent member. That is, in the nappy 1, for example, the laminated nonwoven fabric 10 can be used as the topsheet 21.

In the outer cover 3 of the nappy 1, although the elastic members 71 are disposed between the outer sheet 6 and the inner layer sheet 3i as illustrated in Fig. 7, but the elastic members 71 may not be disposed. In the case of forming an elasticised region without the elastic members 71, an elastic nonwoven fabric that is elastic in the lateral direction Y may be used as a constituent of the outer cover 3.

In the nappy 1, the outer cover 3 has a continuous shape extending across the front region A, the crotch region C, and the rear region B as illustrated in Fig. 7. Alternatively, the outer cover 3 may have a divided shape in which the outer cover 3 is divided into separate members as a front outer cover, a rear outer cover, and a crotch outer cover.

The absorbent article according to the present invention is not limited to a pull-on nappy exemplified by the nappy 1, and is also applicable to all the products for use in absorbing body fluid, specifically, an open type nappy or a sanitary towel, for example.

In addition, the sweat absorbing sheet according to the present invention is not limited to application to the absorbent article described above, and is also applicable to products except the absorbent article. For example, the sweat absorbing sheet according to the present invention can be used as a sheet itself for wiping sweat by a user. In addition, the sweat absorbing sheet is also applicable to insoles for shoe soles, armpit sweat pads, eye masks, face masks, and so forth. When the sweat absorbing sheet according to the present invention is disposed to face the skin of a user, sweat from soles, armpits, or faces can be quickly absorbed and exhaled.

Industrial Applicability

The present invention provides a laminated nonwoven fabric having high absorption performance of body fluid such as sweat and urine, a method for manufacturing the nonwoven fabric, an absorbent article, and a sweat absorbing sheet.

**Claims**

1. A laminated nonwoven fabric (10) having a laminated structure (13) of fiber layers including long fibers, wherein

   the laminated structure (13) includes a first surface (10a) that is a surface of the laminated nonwoven fabric (10) and a second surface (10b) that is another surface of the laminated nonwoven fabric (10),
   the second surface (10b) is constituted by a hydrophobic layer (12) including hydrophobic fibers (15),
   a hydrophilic layer (11) including hydrophilic fibers (14A, 14B) is disposed at the first surface side of the hydrophobic layer (12),
   the hydrophilic layer (11) includes a first hydrophilic layer (11A) and a second hydrophilic layer (11B) disposed in this order from the hydrophobic layer (12),
   the laminated structure (13) includes an interlayer fused portion (16) which has a density higher than those in peripheral portions and in which layers constituting the laminated structure (13) are fused to one another, and
   an inter-fiber distance of constituent fibers of the first hydrophilic layer (11A) is shorter than that of the hydrophobic layer (12);

   **characterized in that**

   the second surface (10b) includes an interlayer fused portion dispersion region in which a plurality of the interlayer fused portions (16) are dispersed in a surface direction,
   in a case where a virtual circle with a radius of 2 mm is disposed at any position in the interlayer fused portion dispersion region of the second surface (10b), a part or a whole of at least one of the interlayer fused portions (16) is included in the virtual circle.

2. The laminated nonwoven fabric according to claim 1, wherein
   an ellipticity of constituent fibers of the first hydrophilic layer (11A) is higher than that of the hydrophobic layer (12).

3. The laminated nonwoven fabric according to claim 1 or 2, wherein

a hydrophilic degree of constituent fibers of the second hydrophilic layer (11B) is higher than that of the first hydrophilic layer (11A), and/or
a fiber diameter of constituent fibers of the second hydrophilic layer (11B) is smaller than that of the first hydrophilic layer (11A), and/or
an inter-fiber distance of constituent fibers of the second hydrophilic layer (11B) is shorter than that of the first hydrophilic layer (11A).

4. The laminated nonwoven fabric according to claim 1 or 2, wherein

a hydrophilic degree of constituent fibers of the second hydrophilic layer (11B) is higher than that of the first hydrophilic layer (11A), and
a fiber diameter of constituent fibers of the second hydrophilic layer (11B) is smaller than that of the first hydrophilic layer (11A).

5. The laminated nonwoven fabric according to claim 1 or 2, wherein

a hydrophilic degree of constituent fibers of the second hydrophilic layer (11B) is higher than that of the first hydrophilic layer (11A), and
an inter-fiber distance of constituent fibers of the second hydrophilic layer (11B) is shorter than that of the first hydrophilic layer (11A).

6. The laminated nonwoven fabric according to any one of claims 1 to 5, wherein the laminated structure includes no high-density fused portion which has a density higher than those in peripheral portions and in which constituent fibers are fused to one another, except for the interlayer fused portion (16).

7. The laminated nonwoven fabric according to any one of claims 1 to 6, wherein the hydrophobic layer (12), the first hydrophilic layer (11A), and the second hydrophilic layer (11B) are joined to one another only by fusion bonding of constituent fibers.

8. A method for manufacturing a laminated nonwoven fabric (10) having a laminated structure (13) of fiber layers including long fibers, the layers constituting the laminated structure (13) being joined to one another in an interlayer fused portion (16), the method comprising:

a base web forming step of spinning a resin to obtain first fibers (41), and depositing the first fibers (41), thereby forming a base web (17);
a first lamination step of spinning a resin to obtain second fibers (42), and depositing the second fibers (42) on the base web (17), thereby forming a first laminated body (18);
a second lamination step of spinning a resin to obtain third fibers (43), and depositing the third fibers (43) on the first laminated body (18), thereby forming a second laminated body (19); and
an interlayer fusing step of heating the second laminated body (19) while partially compressing the second laminated body (19) in a thickness direction, thereby forming the interlayer fused portion (16),
wherein one of the first fibers (41) or the third fibers (43) include hydrophobic fibers (15), whereas the other include hydrophilic fibers (14B), whereas the second fibers (42) include hydrophilic fibers (14A) having a fiber diameter smaller than that of the hydrophobic fibers (15),
wherein the laminated structure (13) includes a first surface (10a) that is a surface of the laminated nonwoven fabric (10) and a second surface (10b) that is another surface of the laminated nonwoven fabric (13),
wherein the first surface (10a) is formed of the deposit of one of the first fibers (41) or the third fibers (43) including the hydrophilic fibers (14B), and the second surface (10b) is formed of the deposit of one of the first fibers (41) or the third fibers (43) including the hydrophobic fibers (15), and
wherein the second surface (10b) includes an interlayer fused portion dispersion region in which a plurality of the interlayer fused portions (16) are dispersed in a surface direction, in a case where a virtual circle with a radius of 2 mm is disposed at any position in the interlayer fused portion dispersion region of the second surface (10b), a part or a whole of at least one of the interlayer fused portions (16) is included in the virtual circle.

9. The method for manufacturing a laminated nonwoven fabric according to claim 8, wherein after deposition of the hydrophilic fibers (14A, 14B), a calendar process is performed on the deposit of the hydrophilic fibers (14A, 14B).

**10.** The method for manufacturing a laminated nonwoven fabric according to claim 8 or 9, wherein the hydrophilic fibers of one of the first fibers (41) or the third fibers (43) have a fiber diameter smaller than that of the second fibers (42).

**11.** The method for manufacturing a laminated nonwoven fabric according to claim 8 or 9, wherein

the hydrophilic fibers are obtained by spinning the resin mixed with a hydrophilising agent, and
a content of the hydrophilising agent included in the second fibers (42) is smaller than a content of the hydrophilising agent in one of the first fibers (41) or the third fibers (43) including hydrophilic fibers.

**12.** The method for manufacturing a laminated nonwoven fabric according to claim 8 or 9, wherein

the hydrophilic fibers of one of the first fibers (41) or the third fibers (43) have a fiber diameter smaller than that of the second fibers (42),
the hydrophilic fibers are obtained by spinning the resin mixed with a hydrophilising agent, and
a content of the hydrophilising agent included in the second fibers (42) is smaller than a content of the hydrophilising agent in one of the first fibers (41) or the third fibers (43) including hydrophilic fibers.

**13.** An absorbent article comprising the laminated nonwoven fabric according to any one of claims 1 to 7, wherein the laminated nonwoven fabric (10) is disposed with the second surface (10b) facing skin of a user.

**14.** Use of the laminated nonwoven fabric according to any one of claims 1 to 7 for absorbing sweat.

**15.** A method for absorbing sweat using the laminated nonwoven fabric according to any one of claims 1 to 7.


**Patentansprüche**

**1.** Laminierter Vliesstoff (10) mit einer laminierten Struktur (13) aus Faserschichten, die lange Fasern umfassen, wobei

die laminierte Struktur (13) eine erste Oberfläche (10a), die eine Oberfläche des laminierten Vliesstoffs (10) ist, und eine zweite Oberfläche (10b) umfasst, die eine andere Oberfläche des laminierten Vliesstoffs (10) ist,
die zweite Oberfläche (10b) aus einer hydrophoben Schicht (12), die hydrophobe Fasern (15) umfasst, ausgebildet ist,
eine hydrophile Schicht (11), die hydrophile Fasern (14A, 14B) umfasst, an einer ersten Oberflächenseite der hydrophoben Schicht (12) angeordnet ist,
die hydrophile Schicht (11) eine erste hydrophile Schicht (11A) und eine zweite hydrophile Schicht (11B) umfasst, die in dieser Reihenfolge von der hydrophoben Schicht (12) angeordnet sind,
die laminierte Struktur (13) einen verschmolzenen Zwischenschichtabschnitt (16) umfasst, der eine höhere Dichte als die in Umfangsabschnitten hat und in dem Schichten, welche die laminierte Struktur (13) bilden, miteinander verschmolzen sind, und
ein Zwischenfaserabstand von Bestandteilfasern der ersten hydrophilen Schicht (11A) kürzer als der der hydrophoben Schicht (12) ist;
**dadurch gekennzeichnet, dass**
die zweite Oberfläche (10b) einen verschmolzenen Zwischenschichtabschnitt-Verteilungsbereich umfasst, in dem mehrere der verschmolzenen Zwischenschichtabschnitte (16) in einer Oberflächenrichtung verteilt sind, in einem Fall, in dem an einer beliebigen Position in dem verschmolzenen Zwischenschichtabschnittbereich der zweiten Oberfläche (10b) ein virtueller Kreis mit einem Radius von 2 mm angeordnet wird, ein Teil oder das Gesamte wenigstens eines der verschmolzenen Zwischenschichtabschnitte (16) in einem virtuellen Kreis enthalten ist.

**2.** Laminierter Vliesstoff nach Anspruch 1, wobei
eine Elliptizität von Bestandteilfasern der ersten hydrophilen Schicht (11A) höher als die der hydrophoben Schicht (12) ist.

**3.** Laminierter Vliesstoff nach Anspruch 1 oder 2, wobei

ein Hydrophilie-Grad von Bestandteilfasern der zweiten hydrophilen Schicht (11B) höher als der der ersten hydrophilen Schicht (11A) ist, und/oder

ein Faserdurchmesser von Bestandteilfasern der zweiten hydrophilen Schicht (11B) kleiner als der der ersten hydrophilen Schicht (11A) ist, und/oder

ein Zwischenfaserabstand von Bestandteilfasern der zweiten hydrophilen Schicht (11B) kürzer als der der ersten hydrophilen Schicht (11A) ist.

4. Laminierter Vliesstoff nach Anspruch 1 oder 2, wobei

ein Hydrophilie-Grad von Bestandteilfasern der zweiten hydrophilen Schicht (11B) höher als der der ersten hydrophilen Schicht (11A) ist, und
ein Faserdurchmesser von Bestandteilfasern der zweiten hydrophilen Schicht (11B) kleiner als der der ersten hydrophilen Schicht (11A) ist.

5. Laminierter Vliesstoff nach Anspruch 1 oder 2, wobei

ein Hydrophilie-Grad von Bestandteilfasern der zweiten hydrophilen Schicht (11B) höher als der der ersten hydrophilen Schicht (11A) ist, und
ein Zwischenfaserabstand von Bestandteilfasern der zweiten hydrophilen Schicht (11B) kürzer als der der ersten hydrophilen Schicht (11A) ist.

6. Laminierter Vliesstoff nach einem der Ansprüche 1 bis 5, wobei die laminierte Struktur, abgesehen von dem verschmolzenen Zwischenschichtbereich (16) keinen verschmolzenen Abschnitt hoher Dichte, der eine höhere Dichte als die in Umfangsabschnitten und Abschnitten, in denen Bestandteilfasern miteinander verschmolzen sind, umfasst.

7. Laminierter Vliesstoff nach einem der Ansprüche 1 bis 6, wobei die hydrophobe Schicht (12), die erste hydrophile Schicht (11A) und die zweite hydrophile Schicht (11B) nur durch Schmelzverbinden von Bestandteilfasern miteinander verbunden sind.

8. Verfahren zur Herstellung eines laminierten Vliesstoffs (10) mit einer laminierten Struktur (13) aus Faserschichten, die lange Fasern umfassen, wobei die Schichten, welche die laminierte Struktur (13) bilden, in einem verschmolzenen Zwischenschichtabschnitt (16) verbunden werden, wobei das Verfahren aufweist:

einen Trägerbahn-Ausbildungsschritt zum Spinnen eines Harzes, um erste Fasern (41) zu gewinnen, und Ablagern der ersten Fasern (41), wodurch eine Trägerbahn (17) ausgebildet wird;
einen ersten Laminierungsschritt zum Spinnen eines Harzes, um zweite Fasern (42) zu gewinnen und Ablagern der zweiten Fasern (42) auf der Trägerbahn (17), wodurch ein erster laminierter Körper (18) ausgebildet wird;
einen zweiten Laminierungsschritt zum Spinnen eines Harzes, um dritte Fasern (43) zu gewinnen und Ablagern der dritten Fasern (43) auf dem ersten laminierten Körper (18), wodurch ein zweiter laminierter Körper (19) ausgebildet wird; und
einen Zwischenschichtverschmelzungsschritt zum Erwärmen des zweiten laminierten Körpers (19), während der zweite laminierte Körper (19) in einer Dickenrichtung teilweise komprimiert wird, wodurch der verschmolzene Zwischenschichtabschnitt (16) ausgebildet wird,
wobei entweder die ersten Fasern (41) oder die dritten Fasern (43) hydrophobe Fasern (15) aufweisen, während die anderen hydrophile Fasern (14B) aufweisen, während die zweiten Fasern (42) hydrophile Fasern (14A) mit einem Faserdurchmesser, der kleiner als der der hydrophoben Fasern (15) ist, umfassen,
wobei die laminierte Struktur (13) eine erste Oberfläche (10a), die eine Oberfläche des laminierten Vliesstoffs (10) ist, und eine zweite Oberfläche (10b), die eine andere Oberfläche des laminierten Vliesstoffs (13) ist, umfasst,
wobei die erste Oberfläche (10a) aus der Ablagerung entweder der ersten Fasern (41) oder der dritten Fasern (43) einschließlich der hydrophilen Fasern (14B) ausgebildet wird, und die zweite Oberfläche (10b) aus der Ablagerung entweder der ersten Fasern (41) oder der dritten Fasern (43) einschließlich der hydrophoben Fasern (15) ausgebildet wird, und
wobei die zweite Oberfläche (10b) einen verschmolzenen Zwischenschichtabschnitt-Verteilungsbereich umfasst, in dem mehrere der verschmolzenen Zwischenschichtabschnitte (16) in einer Oberflächenrichtung verteilt sind, wobei in einem Fall, in dem an einer beliebigen Position in dem verschmolzenen Zwischenschichtabschnittbereich der zweiten Oberfläche (10b) ein virtueller Kreis mit einem Radius von 2 mm angeordnet wird, ein Teil oder das Gesamte wenigstens eines der verschmolzenen Zwischenschichtabschnitte (16) in dem virtuellen Kreis enthalten ist.

**9.** Verfahren zur Herstellung eines laminierten Vliesstoffs nach Anspruch 8, wobei nach der Ablagerung der hydrophilen Fasern (14A, 14B) ein Kalanderverfahren auf der Ablagerung der hydrophilen Fasern (14A, 14B) durchgeführt wird.

**10.** Verfahren zur Herstellung eines laminierten Vliesstoffs nach Anspruch 8 oder 9, wobei die hydrophilen Fasern entweder der ersten Fasern (41) oder der dritten Fasern (43) einen Faserdurchmesser haben, der kleiner als der der zweiten Fasern (42) ist.

**11.** Verfahren zur Herstellung eines laminierten Vliesstoffs nach Anspruch 8 oder 9, wobei

die hydrophilen Fasern durch Spinnen des Harzes, das mit einem hydrophilisierenden Mittel vermischt wird, gewonnen werden, und
ein Gehalt des in den zweiten Fasern (42) enthaltenen hydrophilisierenden Mittels kleiner als ein Gehalt des hydrophilisierenden Mittels entweder in den ersten Fasern (41) oder den dritten Fasern (43), welche die hydrophilen Fasern umfassen, ist.

**12.** Verfahren zur Herstellung eines laminierten Vliesstoffs nach Anspruch 8 oder 9, wobei

die hydrophilen Fasern entweder der ersten Fasern (41) oder der dritten Fasern (43) einen Faserdurchmesser haben, der kleiner als der der zweiten Fasern (42) ist,
die hydrophilen Fasern durch Spinnen des Harzes, das mit einem hydrophilisierenden Mittel vermischt wird, gewonnen werden, und
ein Gehalt des in den zweiten Fasern (42) enthaltenen hydrophilisierenden Mittels kleiner als ein Gehalt des hydrophilisierenden Mittels entweder in den ersten Fasern (41) oder den dritten Fasern (43), welche die hydrophilen Fasern umfassen, ist.

**13.** Absorbierender Artikel, der den laminierten Vliesstoff nach einem der Ansprüche 1 bis 7 aufweist, wobei der laminierte Vliesstoff (10) der Haut eines Benutzers mit der zweiten Oberfläche (10b) zugewandt angeordnet wird.

**14.** Verwendung des laminierten Vliesstoffs nach einem der Ansprüche 1 bis 7 zum Absorbieren von Schweiß.

**15.** Verfahren zum Absorbieren von Schweiß unter Verwendung des laminierten Vliesstoffs nach einem der Ansprüche 1 bis 7.

## Revendications

**1.** Tissu non tissé stratifié (10) qui présente une structure stratifiée (13) de couches de fibres comportant de longues fibres, dans lequel

la structure stratifiée (13) comporte une première surface (10a) qui est une surface du tissu non tissé stratifié (10) et une seconde surface (10b) qui est une autre surface du tissu non tissé stratifié (10),
la seconde surface (10b) est constituée d'une couche hydrophobe (12) comportant des fibres hydrophobes (15),
une couche hydrophile (11) comportant des fibres hydrophiles (14A, 14B) est disposée du côté de la première surface de la couche hydrophobe (12),
la couche hydrophile (11) comporte une première couche hydrophile (11A) et une seconde couche hydrophile (11B) disposées dans cet ordre à partir de la couche hydrophobe (12),
la structure stratifiée (13) comporte une portion fusionnée de couche intermédiaire (16) qui a une densité supérieure à celles des portions périphériques et dans laquelle des couches constituant la structure stratifiée (13) sont fusionnées les unes aux autres, et
une distance entre les fibres de fibres constitutives de la première couche hydrophile (11A) est plus courte que celle de la couche hydrophobe (12) ;
**caractérisé en ce que**
la seconde surface (10b) comporte une région de dispersion de portions fusionnées de couche intermédiaire dans laquelle une pluralité des portions fusionnées de couche intermédiaire (16) sont dispersées dans une direction de surface,
dans le cas où un cercle virtuel d'un rayon de 2 mm est disposé à n'importe quelle position dans la région de dispersion de portions fusionnées de couche intermédiaire de la seconde surface (10b), une partie ou une totalité d'au moins une des portions fusionnées de couche intermédiaire (16) est incluse dans le cercle virtuel.

2. Tissu non tissé stratifié selon la revendication 1, dans lequel une ellipticité de fibres constitutives de la première couche hydrophile (11A) est supérieure à celle de la couche hydrophobe (12).

3. Tissu non tissé stratifié selon la revendication 1 ou 2, dans lequel

   un degré hydrophile de fibres constitutives de la seconde couche hydrophile (11B) est supérieur à celui de de la première couche hydrophile (11A), et/ou
   un diamètre de fibre de fibres constitutives de la seconde couche hydrophile (11B) est plus petit que celui de la première couche hydrophile (11A), et/ou
   une distance entre les fibres de fibres constitutives de la seconde couche hydrophile (11B) est plus courte que celle de la première couche hydrophile (11A).

4. Tissu non tissé stratifié selon la revendication 1 ou 2, dans lequel

   un degré hydrophile de fibres constitutives de la seconde couche hydrophile (11B) est supérieur à celui de de la première couche hydrophile (11A), et
   un diamètre de fibre de fibres constitutives de la seconde couche hydrophile (11B) est plus petit que celui de la première couche hydrophile (11A).

5. Tissu non tissé stratifié selon la revendication 1 ou 2, dans lequel

   un degré hydrophile de fibres constitutives de la seconde couche hydrophile (11B) est supérieur à celui de de la première couche hydrophile (11A), et
   une distance entre les fibres de fibres constitutives de la seconde couche hydrophile (11B) est plus courte que celle de la première couche hydrophile (11A).

6. Tissu non tissé stratifié selon l'une quelconque des revendications 1 à 5, dans lequel la structure stratifiée ne comporte pas de portion fondue à haute densité dont une densité est supérieure à celles des portions périphériques et dans laquelle les fibres constitutives sont fusionnées les unes aux autres, à l'exception de la portion fusionnée de couche intermédiaire (16).

7. Tissu non tissé stratifié selon l'une quelconque des revendications 1 à 6, dans lequel la couche hydrophobe (12), la première couche hydrophile (11A) et la seconde couche hydrophile (11B) sont reliées les unes aux autres uniquement par liaison par fusion de fibres constitutives.

8. Méthode de fabrication d'un tissu non tissé stratifié (10) ayant une structure stratifiée (13) de couches de fibres comportant de longues fibres, les couches constituant la structure stratifiée (13) étant reliées les unes aux autres dans une portion fusionnée de couche intermédiaire (16), la méthode comprenant :

   une étape de formation de bande de base consistant à filer une résine pour obtenir des premières fibres (41), et à déposer les premières fibres (41), formant ainsi une bande de base (17) ;
   une première étape de stratification consistant à filer une résine pour obtenir des deuxièmes fibres (42), et à déposer les deuxièmes fibres (42) sur la bande de base (17), formant ainsi un premier corps stratifié (18) ;
   une deuxième étape de stratification consistant à filer une résine pour obtenir des troisièmes fibres (43), et à déposer les troisièmes fibres (43) sur le premier corps stratifié (18), formant ainsi un second corps stratifié (19) ; et
   une étape de fusion de couche intermédiaire consistant à chauffer le second corps stratifié (19) tout en comprimant partiellement le second corps stratifié (19) dans une direction de l'épaisseur, formant ainsi la portion fusionnée de couche intermédiaire (16),
   dans laquelle les fibres parmi les premières fibres (41) ou les troisièmes fibres (43) comportent des fibres hydrophobes (15), tandis que les autres comportent des fibres hydrophiles (14B) et tandis que les deuxièmes fibres (42) comportent des fibres hydrophiles (14A) ayant un diamètre de fibre plus petit que celui des fibres hydrophobes (15),
   dans laquelle la structure stratifiée (13) comporte une première surface (10a) qui est une surface du tissu non tissé stratifié (10) et une seconde surface (10b) qui est une autre surface du tissu non tissé stratifié (13),
   dans laquelle la première surface (10a) est formée du dépôt de celles des premières fibres (41) ou des troisièmes fibres (43) comportant les fibres hydrophiles (14B), et la seconde surface (10b) est formée du dépôt de celles des premières fibres (41) ou des troisièmes fibres (43) comportant les fibres hydrophobes (15), et
   dans laquelle la seconde surface (10b) comporte une région de dispersion de portions fusionnées de couche intermédiaire dans laquelle une pluralité des portions fusionnées de couche intermédiaire (16) sont dispersées dans une direction de surface, dans le cas où un cercle virtuel d'un rayon de 2 mm est disposé à n'importe quelle position dans la région de dispersion de portions fusionnées de couche intermédiaire de la seconde surface (10b), une partie ou une totalité d'au moins une des portions fusionnées de couche intermédiaire (16) est incluse dans le cercle virtuel.

9. Méthode de fabrication d'un tissu non tissé stratifié selon la revendication 8, dans laquelle, après le dépôt des fibres hydrophiles (14A, 14B), un procédé de calandrage est réalisé sur le dépôt des fibres hydrophiles (14A, 14B).

10. Méthode de fabrication d'un tissu non tissé stratifié selon la revendication 8 ou 9, dans laquelle les fibres hydrophiles des fibres parmi les premières fibres (41) ou les troisièmes fibres (43) ont un diamètre de fibre plus petit que celui des deuxièmes fibres (42).

11. Méthode de fabrication d'un tissu non tissé stratifié selon la revendication 8 ou 9, dans laquelle

les fibres hydrophiles sont obtenues par filage de la résine mélangée à un agent d'hydrophilisation, et
une teneur de l'agent d'hydrophilisation inclus dans les deuxièmes fibres (42) est inférieure à une teneur de l'agent d'hydrophilisation dans celles des premières fibres (41) ou des troisièmes fibres (43) comportant des fibres hydrophiles.

12. Méthode de fabrication d'un tissu non tissé stratifié selon la revendication 8 ou 9, dans laquelle

les fibres hydrophiles des fibres parmi les premières fibres (41) ou les troisièmes fibres (43) ont un diamètre de fibre plus petit que celui des deuxièmes fibres (42),
les fibres hydrophiles sont obtenues par filage de la résine mélangée à un agent d'hydrophilisation, et
une teneur de l'agent d'hydrophilisation inclus dans les deuxièmes fibres (42) est inférieure à une teneur de l'agent d'hydrophilisation dans celles des premières fibres (41) ou des troisièmes fibres (43) comportant des fibres hydrophiles.

13. Article absorbant comprenant le tissu non tissé stratifié selon l'une quelconque des revendications 1 à 7, dans lequel le tissu non tissé stratifié (10) est disposé avec la seconde surface (10b) faisant face à la peau d'un utilisateur.

14. Utilisation du tissu non tissé stratifié selon l'une quelconque des revendications 1 à 7 pour absorber la sueur.

15. Méthode pour absorber la sueur utilisant le tissu non tissé stratifié selon l'une quelconque des revendications 1 à 7.

Fig. 1

Fig. 2

EP 3 597 808 B1

Fig. 3(a) 16

Fig. 3(b) 16

Fig. 3(c) 16

Fig. 3(d) 16

Fig. 3(e) 16

Fig. 3(f) 16

Fig. 3(g) 16

Fig. 3(h) 16

27

Fig. 4

Fig. 5

Fig. 6

Fig. 7

EP 3 597 808 B1

Fig. 8

3i(3)

6R(3)

6(3)

10

21

23

71

22

X

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016112167 A
- JP 2006051649 A
- JP 2002233720 A
- EP 1184020 A2

**Non-patent literature cited in the description**

- JIS Handbook Fibre. Japanese Standards Association, 2000, 764